(19) Europäisches Patentamt
European Patent Office
Office européen des brevets

(11) **EP 2 386 244 B1**

(12) **EUROPÄISCHE PATENTSCHRIFT**

(45) Veröffentlichungstag und Bekanntmachung des Hinweises auf die Patenterteilung:
**13.06.2018   Patentblatt 2018/24**

(51) Int Cl.:
*A61B 3/15* (2006.01)          *A61F 9/008* (2006.01)

(21) Anmeldenummer: **11075184.9**

(22) Anmeldetag: **09.07.2007**

(54) **Ophthalmoskop**

Ophthalmoscope

Ophtalmoscope

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **07.07.2006   EP 06116853**

(43) Veröffentlichungstag der Anmeldung:
**16.11.2011   Patentblatt 2011/46**

(62) Dokumentnummer(n) der früheren Anmeldung(en) nach Art. 76 EPÜ:
**07787243.0 / 2 040 607**

(73) Patentinhaber: **OD-OS GmbH**
**14513 Teltow (DE)**

(72) Erfinder:
• **Liesfeld, Ben**
 **18055 Rostock (DE)**
• **Teiwes, Winfried**
 **14532 Kleinmachnow (DE)**
• **Huppertz, Michael**
 **14163 Berlin (DE)**
• **Amthor, Kay-Uwe**
 **14513 Teltow (DE)**

(74) Vertreter: **Pfenning, Meinig & Partner mbB Patent- und Rechtsanwälte Joachimsthaler Straße 10-12 10719 Berlin (DE)**

(56) Entgegenhaltungen:
WO-A-02/094088      US-B1- 6 267 756
US-B2- 6 758 564

EP 2 386 244 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft ein Ophthalmoskop zur Untersuchung eines Auges eines Patienten.

Derartige optische Geräte werden insbesondere zur Beobachtung des Augenhintergrunds des Patienten eingesetzt, beispielsweise für Netzhautuntersuchungen. Ein Ophthalmoskop muß hochauflösende Farb- bzw. Schwarzweißbilder in kontinuierlicher Abfolge liefern, um zur Diagnose des Auges ebenso wie bei der Durchführung und Dokumentation von therapeutischen Eingriffen eingesetzt werden zu können.

Beispielsweise wird in der Patentschrift US 6 267 756 B1 eine Vorrichtung für die Beobachtung und Behandlung eines Auges mit einem Laser beschrieben. Die Vorrichtung umfasst eine Beleuchtungslichtquelle, deren Licht auf einen zu beobachtenden den Teil des Auges fokussiert wird, eine Detektoreinrichtung für das am Auge reflektierte Licht, einer Auswerte- und Synchronisiereinheit, die aus einem zeitsequentiellen Ausgangssignal der Detektoreinrichtung eine visuelle Darstellung eines zu behandelnden Teils des Auges auf einem Monitor zeigt. Die Vorrichtung umfasst ferner eine Laserbehandlungsvorrichtung, deren Licht auf den zu behandelnden Teil des Auges gerichtet wird. Die Vorrichtung ermöglicht es außerdem, dass auf dem Monitor die visuelle Darstellung des Auges mit einer visuellen Darstellung überlagert wird, in der mindestens eine zu behandelnde Stelle markiert ist, wobei eine Steuereinrichtung den Laser auf die markierte Stelle ausrichtet.

In der Veröffentlichung WO 02/094088 A wird ein Ophthalmoskop zur Untersuchung des Augenhintergrundes eines Patienten mit einer einen Beleuchtungsstrahl erzeugenden Beleuchtungseinrichtung sowie einer der Beleuchtungseinrichtung zuordenbaren Abbildungsoptik, die den Beleuchtungsstrahl auf den Augenhintergrund des Patienten abbildet, einer Beobachtungseinrichtung, in die die Abbildungsoptik einen durch Reflexion des Beleuchtungsstrahls am Augenhintergrund erzeugten Beobachtungsstrahl abbildet sowie einer im Beleuchtungs- und Beobachtungsstrahl eingebrachten Blendenanordnung, deren Blendenspalte variabel änderbar und aufeinander synchron abgestimmt sind.

Es besteht grundsätzlich die Aufgabe, die Dokumentation von am Auge durchgeführten therapeutischen Eingriffen weiter zu verbessern. Erfindungsgemäß wird diese Aufgabe durch ein Ophthalmoskop gemäß dem Patentanspruch 1 gelöst. Bevorzugte Ausführungsformen und Weiterentwicklungen der Erfindung ergeben sich mit den abhängigen Patentansprüchen und mit der nachfolgenden Beschreibung.

Die Abbildung des Augenhintergrunds, beispielsweise der Netzhaut, stellt eine optische Herausforderung dar, denn sowohl die Beleuchtung als auch die Beobachtung des Augenhintergrunds müssen durch eine verhältnismäßig kleine Eintrittspupille des Auges durchgeführt werden. Zudem weist der Augenhintergrund in der Regel nur eine schwache Reflektivität auf, die für rote Farbanteile dominiert, so dass kontrastreiche Farbbilder vom Augenhintergrund in der Regel nur mit einer Lichtquelle mit starken Blau- und Grünanteilen erzeugt werden können.

Die optische Untersuchung des Augenhintergrunds mit Hilfe eines Beleuchtungsstrahls, der nach Reflexion beispielsweise an der Netzhaut als Beobachtungsstrahl untersucht wird, wird zudem durch unerwünschte weitere Reflexionen an den Grenzflächen der Kornea sowie durch unerwünschte Lichtstreuung beispielsweise an Glaskörpertrübungen erschwert. Im nachfolgenden werden all diese störenden Lichtstrahlen, die im Beobachtungsstrahl enthalten sein können, jedoch nicht auf die gewünschte Reflexion des Beleuchtungsstrahls am zu untersuchenden Teil des Auges zurückzuführen sind, zusammenfassend als "Streulicht" bezeichnet.

Zur Lösung dieses Streulichtproblems offenbart die EP 1389943 B1 ein herkömmliches Ophthalmoskop zur Untersuchung eines Auges eines Patienten, umfassend:

eine Beleuchtungsvorrichtung zur Erzeugung eines Beleuchtungsstrahls;
eine Beleuchtungs-Abbildungsoptik zum Abbilden des Beleuchtungsstrahls auf das Auge, und

Mittel zum Scannen des Beleuchtungsstrahls über das Auge, sowie
eine Beobachtungsvorrichtung, die einen elektronischen Sensor mit einem Feld von fotosensitiven Pixeln umfaßt, die jeweils zeilenweise aktivierbar und/oder auslesbar sind;
eine Beobachtungs-Abbildungsoptik zum Abbilden eines durch Reflexion des Beleuchtungsstrahls am Auge erzeugten Beobachtungsstrahls auf die Beobachtungsvorrichtung, und

Mittel zum Ausblenden von Streulicht aus dem Beobachtungsstrahl.

Als Beleuchtungsvorrichtung wird hierbei insbesondere eine Halogenlampe eingesetzt, deren Licht mittels eines Kondensors kollimiert und über die Beleuchtungs-Abbildungsoptik auf den Augenhintergrund des Patienten fokussiert wird. Der am Augenhintergrund reflektierte Beobachtungsstrahl wird über die Beobachtungs-Abbildungsoptik auf eine Bildebene abgebildet, in der sich ein CCD-Sensor als Beobachtungsvorrichtung befindet.

Die Mittel zum Scannen des Beleuchtungsstrahls über das Auge sind bei diesem herkömmlichen Ophthalmoskop durch eine Schlitzblende gebildet, die vor der Halogenlampe in dem vom Kondensor kollimierten Beleuchtungsstrahl oszilliert. Diese Blende ist aus einem lichtundurchlässigen Material, beispielsweise einem Flachblechmaterial, gefertigt und läßt nur einen durch die Größe der Schlitzblende definierten linienförmigen Ausschnitt aus dem Beleuchtungsstrahl durch, der aufgrund der Oszillation der Schlitzblende somit ebenfalls mit dieser Oszillationsfrequenz über das Auge des Patienten hin- und her gescannt wird.

Auch die Mittel zum Ausblenden von Streulicht aus dem Beobachtungsstrahl sind bei diesem herkömmlichen Ophthalmoskop durch eine mechanisch oszillierende Schlitzblende gebildet. Insbesondere schlägt die EP 1389943 B1 vor, die vor der Halogenlampe oszillierende Schlitzblende und die vor dem CCD-Sensor oszillierende Schlitzblende, die aus Abbildungsgründen ohnehin miteinander synchronisiert werden müssen, als Blendenspaltpaar in einem gemeinsamen Flachblech auszubilden.

In der Praxis hat sich hierbei gezeigt, dass insbesondere die Wahl eines vor dem Sensor hin- und her schwingenden Blechs mit einer Schlitzblende zu Problemen führt. Denn einerseits lassen sich die Schwingungen des mechanischen Oszillators für das Blech nur ungenügend vom Gehäuse des Gerätes entkoppeln, so dass allgemein Schwierigkeiten in der Handhabung des herkömmlichen Ophthalmoskops und insbesondere Beeinträchtigungen in der Bildschärfe aufgrund eines Mitvibrierens des Sensors auftreten.

Andererseits sollte zur Erzielung einer möglichst kompakten Ausführung des Ophthalmoskops die Blende im Beobachtungsstrahl möglichst exakt in der Bildebene der Beobachtungsvorrichtung und somit auf dem Sensor liegen. Es muß jedoch ein gewisser Mindestabstand des mechanischen hin- und her schwingenden Blechs vom Sensor eingehalten werden, was unweigerlich zu einer schlechteren Unterdrückung störenden Streulichts führt.

Es ist daher nützlich, ein herkömmliches Ophthalmoskop derart weiterzuentwickeln, dass die Zahl mechanisch schwingender Bauteile verringert wird, insbesondere in der Nähe der Beobachtungsvorrichtung.

Dies kann dadurch erreicht werden, dass die Mittel zum Ausblenden von Streulicht aus dem Beobachtungsstrahl eine elektronische Steuerschaltung zum Auslesen wenigstens einer Pixelzeile des Sensors umfassen, und dass das Ophthalmoskop eine elektronische Kontrolleinheit umfaßt, die dazu ausgelegt ist, die Mittel zum Scannen und die elektronische Steuerschaltung derart zu kontrollieren, dass das Scannen des Beleuchtungsstrahls über das Auge synchron mit der Änderung der aktuell auszulesenden Pixelzeile erfolgt.

Derartige elektronische Sensoren, insbesondere CMOS-Sensoren, sind im Stand der Technik an sich bekannt, vgl. beispielsweise die WO 99/05853. Der Einsatz eines derartigen elektronischen Sensors bei einem Ophthalmoskop erlaubt es nun erstmals, die im Stand der Technik benutzte mechanische Blende gleichsam durch eine elektronische Blende ("rolling shutter") zu ersetzen. Denn mit Hilfe der elektronischen Steuerschaltung können alle Pixelzeilen eines derartigen Sensors gezielt aktiviert, deaktiviert und ausgelesen werden. Im aktivierten Zustand eines Pixels führt Lichteinfall zur kontinuierlichen Erzeugung von Ladungen, die bei einem CMOS-Sensor über eine dem jeweiligen Pixel zugeordnete Verstärkerelektronik in ein Spannungssignal umgesetzt werden. Im deaktivierten Zustand hingegen unterbleibt ein derartiger Spannungsaufbau, das Pixel oder auch die gesamte Pixelzeile ist gleichsam "abgeschaltet".

Durch gezieltes Aktivieren wenigstens einer Pixelzeile in einem bestimmten Bereich des Felds von fotosensitiven Pixeln und gleichzeitiges Deaktivieren aller übrigen Pixelzeilen kann somit auf elektronische Weise das gleiche Ergebnis wie mit Hilfe einer mechanischen Schlitzblende erzielt werden. Auf diese Weise kann Streulicht aus dem Beobachtungsstrahl ausgeblendet werden, ohne dass hierfür ein mechanisch schwingendes Blech mit einer Schlitzblende vor der Beobachtungsvorrichtung erforderlich ist. Zudem befindet sich die hier vorgeschlagene elektronische Blende im Gegensatz zur mechanischen Blende beim herkömmlichen Ophthalmoskop nicht vor, sondern in der Bildebene der Beobachtungsvorrichtung, was die Streulichtunterdrückung verbessert.

Zweckmäßigerweise ist vorgesehen, dass die elektronische Steuerschaltung dazu ausgelegt ist, jeweils eine einzelne Pixelzeile auszulesen. Auf diese Weise läßt sich eine äußerst feine Auflösung des elektronischen Sensors erzielen, die bei typischen CMOS-Sensoren einer Höhe von ca. $5\,\mu m$ entspricht. Es wäre jedoch grundsätzlich auch vorstellbar, mit Hilfe der elektronischen Steuerschaltung mehrere Pixelzeilen, insbesondere benachbarte Pixelzeilen, zusammenzufassen, d.h. stets gemeinsam zu aktivieren, zu deaktivieren oder auszulesen, und somit unter Verschlechterung der Auflösung die Lichtausbeute zu verbessern.

Zweckmäßigerweise ist die elektronische Steuerschaltung dazu ausgelegt, jeweils nach Ablauf einer einstellbaren Zeitverzögerung ($\Delta t$) die aktuell auszusende Pixelzeile zu ändern, insbesondere von einer Pixelzeile zu einer benachbarten Pixelzeile. In der bevorzugten Ausführungsvariante, bei der unmittelbar benachbarte Pixelzeilen nacheinander ausgelesen werden, benötigt die elektronische Steuerschaltung somit für einen vollständigen Durchlauf, d.h. ein vollständiges Auslesen des elektronischen Sensors mit N Pixelzeilen, insgesamt eine Zeitdauer $T=N\times\Delta t$. Hierbei kann der Parameter N, der die Zahl der Pixelzeilen des Sensors beschreibt, ausgehend von einer durch die Bauart des Sensors vorgegebenen Obergrenze durch das oben beschriebene elektronische Zusammenfassen von benachbarten Pixelzeilen reduziert werden, während gleichzeitig der Zeitverzögerungsparameter $\Delta t$ vom Bediener des Ophthalmoskops frei einstellbar ist. Auf diese Weise kann die für einen vollständigen Auslese-Durchlauf benötigte Gesamtzeitdauer eingestellt werden, entsprechend der Einstellung der Schwingungsperiodendauer bzw. der Schwingungsfrequenz der beim herkömmlichen Ophthalmoskop vorgesehenen mechanischen Schlitzblende.

In einer bevorzugten Ausführungsform der Erfindung ist die elektronische Steuerschaltung ferner dazu ausgelegt, jede Pixelzeile während einer einstellbaren Belichtungszeit ($t_{INT}$) vor ihrem Auslesen zu aktivieren. Durch Einstellen dieser Belichtungszeit $t_{INT}$ als Vielfaches der Zeitverzögerung $\Delta t$ läßt sich somit erreichen, dass jede Pixelzeile während eines längeren Zeitraums $t_{INT}$ belichtet wird, während dessen sich ihr die aktuell auszulesende Pixelzeile nähert. Auf diese Weise kann die effektive Breite der elektronischen Spaltblende flexibel eingestellt werden.

Zweckmäßigerweise ist die elektronische Steuerschaltung dazu ausgelegt, den Auslesevorgang infolge eines externen Triggersignals zu starten. Auf diese Weise kann die auch bei der hier beschriebenen Weiterentwicklung erforderliche Synchronisierung der Blende für die Beobachtungsvorrichtung mit den Mitteln zum Scannen des Beleuchtungsstrahls sichergestellt werden. Beispielsweise kann ein zentraler Steuercomputer für das Ophthalmoskop einerseits mit Hilfe dieses Triggersignals die elektronische Steuerschaltung des elektronischen Sensors und andererseits einen Antrieb für eine mechanische Schlitzblende im Beleuchtungsstrahl kontrollieren.

Grundsätzlich läßt sich mit Hilfe eines derartigen zentralen Steuercomputers eine Synchronisierung der elektronischen Blende im Beobachtungsstrahl mit einer mechanischen Blende im Beleuchtungsstrahl problemlos erzielen. Der zentrale Steuercomputer kann selbstverständlich auch Teil des elektronischen Sensors oder Teil der Beleuchtungsvorrichtung sein.

Um nun jedoch auch im Beleuchtungsstrahl eine mechanisch schwingende Schlitzblende überflüssig zu machen, kann in einer vorteilhaften Weiterbildung vorgesehen sein, dass die Mittel zum Scannen des Beleuchtungsstrahls über das Auge einen schwenkbar gelagerten Spiegel mit einer Scanvorrichtung umfassen. Um sicherzustellen, dass der auf den schwenkbar gelagerten Spiegel auftreffende Beleuchtungsstrahl bereits die gewünschte Linienform besitzt, kann entweder vorgesehen sein, dass es ferner eine feste Spaltblende zum Auskoppeln eines linienförmigen Beleuchtungsstrahls umfaßt, oder es ferner eine Linienfokussieroptik zum Fokussieren des Beleuchtungsstrahls in einer Linie umfaßt, wobei in diesem letztgenannten Fall zweckmäßigerweise die Linienfokussieroptik eine Zylinderlinse umfaßt. Die Verwendung eines derartigen schwenkbar gelagerten Spiegels, der mit Hilfe eines Galvanometerantriebs angetrieben und bei einem Ophthalmoskop eingesetzt wird, um einen von einer Linienfokussieroptik gelieferten Beleuchtungsstrahl zu scannen, ist aus der US 6,758,564 B2 grundsätzlich bekannt, auf die insofern verwiesen wird.

Für eine zentrale Kontrolle der wesentlichen Bauteile der vorgeschlagenen Weiterentwicklung des Ophthalmoskops ist zweckmäßigerweise vorgesehen, dass die elektronische Kontrolleinheit dazu ausgelegt ist, die elektronische Steuerschaltung des elektronischen Sensors und die Scanvorrichtung des schwenkbaren Spiegels zu kontrollieren. Die elektronische Kontrolleinheit kann dann durch Senden des oben besprochenen Triggersignals an die elektronische Steuerschaltung des elektronischen Sensors sicherstellen, dass bei einem beispielsweise sinusförmig schwingenden Spiegel der Auslesevorgang im elektronischen Sensor zu einem geeigneten Zeitpunkt startet, beispielsweise durch ein Auslesen der oberen Pixelzeile des elektronischen Sensors, sobald der schwingende Spiegel seinen oberen Umkehrpunkt erreicht hat.

Bei dieser Weiterbildung des Ophthalmoskops ist die elektronische Kontrolleinheit dazu ausgelegt, die Scanvorrichtung und die elektronische Steuerschaltung derart zu kontrollieren, dass das Scannen des Beleuchtungsstrahls über das Auge synchron mit der Änderung der aktuell auszulesenden Pixelzeile erfolgt. Beispielsweise kann die elektronische Kontrolleinheit der Scanvorrichtung eine Sägezahntrajektorie vorgeben, die exakt der Bewegung des elektronischen Spalts im elektronischen Sensor entspricht.

Für besonders präzise Untersuchungen des Auges des Patienten mit besonders hoher Ortsauflösung ist vorgesehen, dass die Stärke der Linie des fokussierten Beleuchtungsstrahls der Höhe einer Pixelzeile des elektronischen Sensors entspricht. Man erzielt hierdurch eine konfokale Abbildung des Auges, wodurch sich dreidimensionale Informationen über das beobachtete Objekt gewinnen lassen.

Bei Verwendung der besprochenen Linienfokussieroptik kann die Beleuchtungsvorrichtung zweckmäßigerweise ein Laser sein. Alternativ kann jede andere Form einer im wesentlichen punktförmigen Beleuchtungsvorrichtung eingesetzt werden, beispielsweise ein Ende eines beleuchteten Lichtwellenleiters.

In einer Weiterbildung der Erfindung ist vorgesehen, dass die Beobachtungsvorrichtung zwei Sensoren zur stereoskopischen Untersuchung des Auges umfaßt. Bei einer derartigen Ausführungsform werden die Vorteile der Weiterentwicklung besonders deutlich, da für eine stereoskopische Betrachtung mit zwei Beobachtungsvorrichtungen im Stand der Technik insgesamt sogar drei hin-und her oszillierende Schlitzblenden erforderlich sind, auf die erfindungsgemäß weitgehend bzw. bei Verwendung der Linienfokussieroptik sogar vollständig verzichtet werden kann.

Ferner ist in einer weiteren Ausführungsform der Erfindung vorgesehen, dass sie eine separate Lichtquelle und einen separaten Bildsensor zur Untersuchung des Auges umfaßt. Insbesondere kann hierbei mit Hilfe der Beleuchtungsvorrichtung, der Beleuchtungs-Abbildungsoptik, der Beobachtungs-Abbildungsoptik und der Beobachtungsvorrichtung der Augenhintergrund untersucht werden, beispielsweise die Netzhaut des Auges, während die zusätzliche Abbildungseinheit zur Untersuchung des Augenvordergrunds dient. Dies kann sowohl der medizinischen Untersuchung des Augenvordergrunds selbst dienen, als auch der automatisierten Positionierung des Auges des Patienten, als auch einer Verfolgung von Augenbewegungen (Tracking) des gesamten Auges anhand der Informationen, die man beispielsweise durch Reflexion eines Lichtstrahls am Augenvordergrund erhält. Insbesondere beim Aufsummieren von Augenhintergrund-Bildern bei Angiographie ist es sinnvoll, mittels einer derartigen gleichzeitigen Beobachtung des Augenvordergrunds Augenbewegungen feststellen zu können, die sich zwischen den einzelnen Bildern des Augenhintergrunds ereignet haben.

Wie eingangs erwähnt, ist das erfindungsgemäße Ophthalmoskop um Therapiefunktionen erweitert, wie nachfolgend erläutert werden wird:

Eine wichtige Therapieform in der Augenheilkunde zur Behandlung von Krankheiten wie Diabetischer Retinopathie oder altersbedingter Makuladegeneration ist die thermische Einwirkung durch Licht auf zu behandelnde Gewebebereiche, wie z. B. die photodynamische Therapie (PDT), die Photokoagulation von Gewebe oder neuere Verfahren wie selektive Retina-Therapie (SRT) und Therapie unterhalb der Koagulationsschwelle (Sub-Threshold Therapy, STT). Bei allen diesen Therapie-Formen wird ein intensiver Lichtimpuls gebündelt auf ein spezielles Gebiet der Netzhaut geleitet. Bei der Anwendung der Therapie ist darauf zu achten, dass wichtige Gebiete des Augenhintergrundes (z. B. die Makula) nicht versehentlich geschädigt werden.

Der behandelnde Arzt benötigt daher bei dieser Art des therapeutischen Eingriffs neben einem Live-Bild vom Augenhintergrund eine sehr präzise Kontrolle über die Position des Therapie-Lichtstrahls. Als Hilfsmittel zur Positionierung des Therapie-Lichtstrahls wird typischerweise ein Zielstrahl dem Therapie-Lichtstrahl koaxial überlagert. Dieser ist für den Arzt immer sichtbar, besitzt aber eine andere Wellenlänge und eine weit geringere Intensität als der Therapie-Lichtstrahl und führt daher nicht zu Veränderungen des zu Augenhintergrundes. Reflexe von den verschiedenen Grenzflächen des Auges, die durch die Lichtquelle zur Beleuchtung des Augenhintergrundes wie auch durch den Zielstrahl erzeugt werden, erschweren die Kontrolle jedoch erheblich.

Ein Ophthalmoskop, das ein reflexfreies Live-Bild des Augenhintergrundes erzeugt und gleichzeitig einen Therapiestrahl, dem i. d. R. ein Zielstrahl anderer Wellenlänge überlagert ist, auf den Augenhintergrund projiziert, stellt ein wichtiges Instrument für die Augenheilkunde dar. Es ermöglicht die Unterstützung des Arztes durch den Computer z. B. bei der Navigation über den Augenhintergrund zur Positionierung der Photokoagulationspunkte. Eine solche Computer-Unterstützung ist in anderen Bereichen der Medizin (bspw. der minimalinvasiven Chirurgie oder der Neurochirurgie) bereits Stand der Technik. In der Augenheilkunde scheitert ihre Implementierung bisher an den mangelhaften Beobachtungs- und Behandlungsinstrumenten.

Es ist möglich, diese technische Lücke mit einem Ophthalmoskop hier beschriebener Art zu schließen.

Durch die Computer-Unterstützung wird der therapeutische Eingriff objektiv dokumentiert. Bisher ist dies nur näherungsweise möglich, indem z. B. bei der Photokoagulation die Netzhaut durch den Lichtimpuls derart stark erhitzt wird, dass eine dauerhafte und deutlich sichtbare Veränderung des Gewebes entsteht. Dies führt dazu, dass die zerstörten Gewebeareale und damit der Verlust an Sehkraft nach der Therapie, meist viel größer sind als medizinisch notwendig. Moderne Behandlungsverfahren wie PDT, SRT und STT zielen gerade darauf, sichtbare Schädigungen der Netzhaut zu vermeiden. Ohne die mit der vorliegenden Erfindung mögliche Dokumentationsverfahren ist eine Qualitätssicherung bei diesen Therapien nicht möglich.

Typischerweise wird für die Behandlung der Netzhaut mit intensiven Lichtpulsen eine Spaltlampe verwendet, auf die ein Photokoagulationslaser mit einer entsprechenden Einkopplungsoptik adaptiert ist. Eine Spaltlampe ist ein Stereo-Mikroskop mit einer variablen Spaltbeleuchtung, wobei das Mikroskop wie auch die Beleuchtung um eine gemeinsame Achse in einer Ebene drehbar gelagert sind.

Zur Beobachtung des Augenhintergrundes hält der Benutzer mit der Hand eine ophthalmoskopische Linse oder ein Kontaktglas vor bzw. auf das Auge des Patienten. Das Therapielasersystem besteht meist aus der koaxialen Kombination aus Zielstrahl und Therapiestrahl, die zweckmäßigerweise, um ihre Unterscheidung zu erleichtern, verschiedene Wellenlängen haben. Der Therapiestrahl wird i. d. R. zwischen Mikroskop und ophthalmoskopischer Linse koaxial zum Beobachtungsstrahlengang eingekoppelt.

Es ist daher unvermeidbar, dass insbesondere der Zielstrahl auf den verschiedenen Grenzflächen zwischen Spaltlampe und Augenhintergrund starke Reflexe erzeugt, die vom Beobachter als störend wahrgenommen werden. Diese Reflexe sind sehr viel heller als das vom Augenhintergrund reflektierte Licht und können vom Betrachter nur mühsam durch geschicktes Bewegen/Ausrichten von Mikroskop und ophthalmoskopischer Linse oder Kontaktglas an den Rand des Bildfeldes gebracht werden, bzw. sie müssen einfach vom Betrachter toleriert werden. Reflexionen an Grenzschichten des menschlichen Auges sind grundsätzlich technisch (d. h. durch Vergütung der Oberflächen) nicht vermeidbar.

Die genannten Reflexe des Zielstrahls behindern jedoch nicht nur die Arbeit des Benutzers, der durch Okulare des Mikroskops schaut, sondern erschweren oder verhindern auch ggf. den Einsatz von Systemen, die Bilder des Augenhintergrundes mit Hilfe elektronischer Sensoren aufnehmen und den Benutzer während der Therapie durch computergestützte Auswertung unterstützen sollen.

Mit einem Ophthalmoskop hier vorgeschlagener Art ist es prinzipiell möglich, die bestehenden Einschränkungen/Nachteile des Standes der Technik zu beseitigen, indem sie in einer weiteren Ausführungsform eine Vorrichtung zur Projektion eines Ziel- und Therapiestrahls bietet und die reflexfreie Beobachtung des Augenhintergrundes mit dem auf ihn projizierten Zielstrahl ermöglicht. Es wird ein Zielstrahl eingesetzt, der nicht polarisiert sein muss, was die Kosten für die Lichtquelle und die verwendete Optik reduziert. Reflexe, die durch den Zielstrahl hervorgerufen werden könnten, werden vollständig unterdrückt. Dabei ist nur ein einziger Detektor zur Verfolgung des Zielstrahls auf dem Objekt nötig.

Der Therapiestrahl kann mit dem ihm überlagerten Zielstrahl mit Hilfe eines Strahlteilers in den Strahlengang des hier vorgeschlagenen Instruments eingekoppelt werden. Dies kann auf vielfältige Art und Weise erfolgen und ist z. T. in der EP 1389943 B1 (Abb. 3-5) offenbart. Der Therapiestrahl kann dabei koaxial zum Beleuchtungsstrahlengang oder koaxial zur Hauptinstrumentenachse angeordnet sein. Er darf jedoch nicht koaxial zum Beobachtungsstrahlengang liegen, da dies dem angewandten Prinzip zur Reflexunterdrückung widerspricht, das auf einer Trennung der Strahlengänge in der Pupillenebene des Auges beruht. Die Trennung der Strahlengänge führt dazu, dass das Bild eines durch den Zielstrahl an einer optischen Grenzschicht zwischen Sensor- und Objektebene hervorgerufenen Reflexes nicht an der gleichen Position auf dem Sensor entsteht wie das Bild des auf dem Objekt (hier: der Augenhintergrund) erzeugten Zielstrahlflecks.

Der Strahlteiler kann zweckmäßigerweise hochreflektiv für die Wellenlänge des Therapiestrahls, teilweise jedoch durchlässig für die Wellenlänge des Zielstrahls ausgeprägt sein. Dadurch erreicht nur Licht des Zielstrahls und nicht des Therapiestrahls den Detektor. Alternativ kann der Strahlteiler wellenlängenunabhängig ausgeführt sein und im Beobachtungsstrahlengang ein zusätzlicher selektiver Filter eingesetzt werden, der das Licht des Therapiestrahls unterdrückt. Unerwünschte Reflexe und Streulicht, die durch den Zielstrahl verursacht werden, können dadurch unterdrückt werden, dass der Zielstrahl in einer Weise gepulst betrieben wird, die im Folgenden beschrieben wird.

Betrachtet man einen festen Punkt auf dem Objekt, auf den der Zielstrahl gerichtet ist, wird der Zielstrahl erfindungsgemäß nur zu genau dem Zeitpunkt $T_2$ für die Zeitdauer $T_p$ eingeschaltet, wenn die Beobachtungsblende des konfokalen Abbildungssystems genau diesen Objektpunkt überstreicht. Die Synchronisation der Aktivierung des Zielstrahls mit der konfokalen Blendenanordnung kann erfindungsgemäß durch eine Steuereinheit hergestellt werden. Diese Steuereinheit verfügt über Informationen über die jeweilige Position des Zielstrahlflecks auf dem Objekt und über den Startzeitpunkt der Blendenbewegung. Vorzugsweise ist diese Steuereinheit mit der Steuereinheit, die die Synchronisation von Beleuchtungs- und Beobachtungsblendenbewegung herstellt, identisch.

Dies kann beispielsweise dadurch realisiert sein, dass die Steuereinheit ein analoges oder digitales Positionssignal der Steuerspiegel des Zielstrahls sowie einen Synchronisationsimpuls der Blendenbewegung empfängt. Die Position des Zielstrahlflecks auf dem Objekt kann z. B. über die Position eines oder mehrerer Umlenkspiegel, die die Winkelauslenkung des Zielstrahls bestimmen, a priori bekannt sein. Aus der Position des Zielstrahlflecks, (x, y), und der Geschwindigkeit der Blendenbewegung, v, läßt sich eine zeitliche Verzögerung $\tau=y/v$ (bei Bewegung der Blende in y-Richtung) zwischen dem Startzeitpunkt der Blendenbewegung und der Aktivierung des Zielstrahls berechnen und in eine entsprechende zweckmäßigerweise rechteckförmige Pulsfolge umsetzen. Wird der Zielstrahl auf dem Objekt bewegt, wird die zeitliche Verzögerung entsprechend der Position angepaßt.

Die erfindungsgemäße Vorrichtung stellt minimale Anforderungen an die verwendete Therapie- und Zielstrahlquelle. Die verwendeten Quellen müssen nicht notwendigerweise polarisiert sein und der Pulsbetrieb, der erfindungsgemäß auf einer Zeitskala von $100\mu s$... 10ms stattfindet, ist an sich bekannt.

Erfindungsgemäß wird eine Steuereinheit eingesetzt, die die durch das erfindungsgemäße Instrument mit dem Sensor aufgenommenen Bilder insbesondere in Echtzeit auswertet. Diese Steuereinheit führt folgende Funktionen aus:

- Erkennung bzw. Bestimmung eines auf dem Objekt ortsfesten, daher objektbezogenen Koordinatensystems anhand von Strukturen auf dem Objekt in den von der erfindungsgemäßen Vorrichtung aufgenommenen Bildern bzw. auf Bildern desselben Objektes, die mit anderen Vorrichtungen aufgenommen wurden.

Die Steuereinheit kann außerdem eine oder mehrere der folgenden Funktionen ausführen:

- Bestimmung der Position des Zielstrahls in diesem objektbezogenen Koordinatensystem,

- Positionierung des Zielstrahlflecks auf dem Augenhintergrund nach Vorgabe des Benutzers über eine geeignete Schnittstelle (z. B. einen Joystick) durch einen oder mehrere geeignete durch die Steuereinheit veränderliche Umlenkspiegel,

- Positionierung des Zielstrahlflecks ohne direkte Einwirkung des Benutzers durch einen oder mehrere geeignete durch die Steuereinheit veränderliche Umlenkspiegel,

- Freigabe bzw. Unterbindung der Auslösung eines Therapie-Lichtimpulses,

- Steuerung der Parameter des Therapie-Lichtimpulses (wie z. B. im Falle der Photokoagulation der Pulsdauer, Pulswiederholrate, Lichtleistung etc.),

- Messung der Dauer, Intensität und Häufigkeit der Therapie-Lichtimpulse,

- Speicherung der Steuerungsparameter der Therapie-Lichtimpulse und Speicherung der gemessenen Werte.

Im Zusammenspiel mit einer geeigneten Anzeige (z. B. einem Bildschirm) des erfindungsgemäßen Ophthalmoskops werden folgende Verfahren möglich:

- Die Steuereinheit überlagert Informationen auf die Anzeige des Live-Bildes, wobei die Transformationen der Position der überlagerten Informationen in Bezug auf das Live-Bild kompensiert werden, d. h. sowohl der Position der überlagerten Informationen wie auch dem Live-Bild liegt das gleiche Objekt-bezogene Koordinatensystem zugrunde. Die Überlagerung kann durch schnellen Bildwechsel oder durch kontrastbasiertes Überblenden erfolgen. Die Informationen bestehen aus Bildern des Objektes, die zu einem früheren Zeitpunkt oder unter Verwendung anderer Bildgebungsverfahren mit einem anderen Instrument aufgenommen wurden. Prinzipiell wäre es aber auch möglich, dass die Informationen aus vom Benutzer zu einem anderen Zeitpunkt erstellten graphischen Darstellungen im Objektkoordinatensystem bestehen.

- Während der Durchführung einer therapeutischen Maßnahme mittels des Therapielichtstrahls führt die Steuereinheit eine Speicherung der Einwirkungsorte (der Koordinaten im objektbezogenen Koordinatensystem, an denen Lichtimpulse appliziert wurden) und der Parameter der Therapie-Lichtimpulse (wie Pulsdauer, Lichtleistung etc.) durch, so dass diese Informationen auch ohne sichtbare Spuren der Lichteinwirkung auf dem Objekt jederzeit durch Überlagerung der gespeicherten Informationen wieder auf Live- oder Standbildern sichtbar gemacht werden können.

Außerdem sind folgende weitere Verfahren möglich:

- Der Benutzer definiert Bereiche (Zonen) im objektbezogenen Koordinatensystem auf dem Objekt, in denen die Applikation von Therapie-Lichtimpulsen durch die Steuereinheit generell zugelassen oder generell unterbunden wird. Dies kann anhand eines mit dem erfindungsgemäßen Gerät zuvor erfassten Einzelbildes oder auch unter Verwendung von Bildern anderer Geräte nach dem Stand der Technik erfolgen. Die Steuereinheit bestimmt zu diesem Zweck in Echtzeit die Position des Zielstrahls im objektgebundenen Koordinatensystem und vergleicht die Position mit den vorgegebenen Bereichen und erteilt ggf. eine Freigabe für bzw. unterbindet die Auslösung eines Therapie-Lichtimpulses. In einer weiteren Ausführungsform führt die Steuereinheit die Definition der o. g. Zonen durch analyse des Bildes automatisch aus.

- Der Benutzer legt auf einem Referenzbild einen oder mehrere Orte im objektbezogenen Koordinatensystem fest (Zielpositionen), auf dem ein Therapie-Lichtimpuls appliziert werden soll. Die Steuereinheit unterstützt daraufhin die Applikation eines Therapie-Lichtimpulses auf eine vorgegebene Position im objektbezogenen Koordinatensystem des Objektes.

  ∘ Während der Benutzer die Position des Zielstrahls manuell verändert zeigt die Steuereinheit die Zielposition im objektbezogenen Koordinatensystem als überlagerte Information im Live-Bild an.

  ∘ Die Steuereinheit gibt genau dann die Auslösung eines Therapie-Lichtimpulses frei, wenn der Benutzer den Zielstrahl mit einer a priori festgelegten Genauigkeit auf die Zielposition gesteuert hat.

  ∘ Die Steuereinheit löst genau dann selbsttätig einen Lichtimpuls aus, wenn sie den Zielstrahl selbsttätig auf die Zielposition im objektbezogenen Koordinatensystem gesteuert hat.

- Die Steuereinheit erzeugt ein Muster aus zwei oder mehreren Zielstrahlflecken auf dem Augenhintergrund, indem es den Zielstrahl in einer geeigneten zur Bewegung der Beobachtungsblende synchronisierten Weise bewegt und aktiviert. Die Position des Musters auf dem Augenhintergrund wird durch den Benutzer über eine Schnittstelle bestimmt oder durch die Steuereinheit ohne Einwirkung des Benutzers automatisch gesetzt. Die Auslösung des Therapie-Lichtimpulses erfolgt daraufhin auf den Positionen des durch den Zielstrahl erzeugten Musters. Eine Applikation von Therapie-Lichtimpulsen in solchen Mustern erhöht die Regelmäßigkeit der Impuls-Plazierung auf dem Augenhintergrund und damit die Reproduzierbarkeit des Therapieergebnisses. In einer weiteren Ausformung der Erfindung wird ein Muster nicht real auf dem Augenhintergrund erzeugt, sondern nur virtuell dem angezeigten Live-Bild überlagert. Die Bewegung des virtuellen Musters ist dabei an die Bewegung des Zielstrahlflecks auf dem Augenhintergrund geknüpft.

- Die Steuereinheit führt eine Stabilisierung des Live-Bildes bzgl. des Koordinatensystems des Objektes und der Anzeige aus, d. h. das Objekt-Koordinatensystem wird mit Bezug auf das Koordinatensystem der Anzeige fest gehalten.

- Die Steuereinheit führt eine Stabilisierung des Zielstrahlflecks im Objekt-Koordinatensystem aus, d. h. die Steuereinheit bewegt den Zielstrahl in einer geeigneten Weise, so dass er die gleichen Relativbewegungen ausführt wie das Objekt.

- Die Steuereinheit führt eine gleichzeitige Stabilisierung des Live-Bildes zum Koordinatensystem der Anzeige und die Stabilisierung des Zielstrahlflecks im Objekt-Koordinatensystem durch.

- Nach der Applikation eines Therapie-Lichtimpulses wertet die Steuereinheit die Reflektivität des Gewebes an der Stelle der Therapie-Lichteinwirkung mittels Bildverarbeitung oder eine andere Messgröße aus, die mit der Temperatur des Gewebes während der Therapie-Lichtimpulseinwirkung und damit dem therapeutischen Effekt korreliert ist, und verändert die Parameter der Therapie-Lichtquelle für den nächsten Therapie-Lichtimpuls in geeigneter Weise. In einer weiteren Ausführung wird die Auswertung mittels Bildverarbeitung oder die Auswertung einer geeigneten Messgröße während der Applikation eines Therapie-Lichtimpulses ausgeführt und die Therapie-Lichtimpulseinwirkung beendet, sobald ein angestrebter Zustand des Gewebes erreicht ist.

Das erfindungsgemäße Ophthalmoskop läßt sich zusätzlich oder alternativ zu den oben beschriebenen Therapiefunktionen auch hinsichtlich der Durchführung von Fluoreszenzmessungen erweitern, wie nachfolgend erläutert werden wird:
Neben der Erzeugung von einfachen Farbbildern der Netzhaut werden Ophthalmoskope auch für komplexere Bildgebungsverfahren verwendet wie z. B. für den qualitativen Nachweis der Fluoreszenz eines Farbstoffes, der in die Blutbahn des Patienten eingebracht wurde, in den Gefäßen der Netzhaut. Die besondere Herausforderung auf diesem Gebiet besteht im Nachweis der schwachen Fluoreszenz mit einer Optik, deren Lichtleitwert durch die besonderen Gegebenheiten bei der Beobachtung des Augenhintergrundes begrenzt ist. Eine besondere Herausforderung besteht darin, die Fluoreszenz von natürlicherweise im menschlichen Auge vorhandenen Farbstoffen (z. B. Lipofuscin) und ihre räumliche Verteilung nachzuweisen, deren Konzentration und damit deren Leuchtkraft nach Fluoreszenzanregung i. d. R. sehr gering ist.

In der Regel werden für eine Fluoreszenz-Messung mit einer breitbandigen Lichtquelle zwei Filter verwendet: ein Anregungsfilter für die Beleuchtung und ein Sperrfilter für die Beobachtung. Bei Verwendung einer schmalbandigen Lichtquelle (z. B. Laser, LED) kann auf den Anregungsfilter verzichtet werden. Die Transmissionskurven der Filter sind derartig ausgeführt, dass ihre maximale Transmission mit den Maxima der Absorptions- bzw. Emissionskurven der Farbstoffe übereinstimmt. Gleichzeitig muß der Sperrfilter jedoch effizient das gesamte Licht der Anregung unterdrücken, um ein hohes Signal-zu-Rausch-Verhältnis zu erhalten.

Das Fluoreszenzsignal eines Objektes ist in der Regel um Größenordnungen kleiner als z. B. das vom Objekt nach dem Lambertschen Gesetz gestreute Anregungslicht. Ein schwaches Meßsignal erfordert teure, empfindliche Sensoren, die dadurch bedingt eine niedrige Auflösung haben. Auf der anderen Seite kann die Lichteinstrahlung auf das Objekt gerade im Bereich der Augenheilkunde nicht beliebig erhöht werden, es bestehen vielmehr gerade bei kurzen sichtbaren Wellenlängen empfindlich niedrige Grenzwerte. Daher kann nicht auf diesem Weg das Meßergebnis verbessert werden.

Die in der Bildverarbeitung übliche Methode, das Signal-zu-Rausch-Verhältnis durch Mittelung mehrerer Bilder (Messungen) zu erhöhen, kann in der Augenheilkunde im Allgemeinen nicht angewendet werden, da sich das Objekt, das menschliche Auge, während der Beobachtung bewegt. Die Registrierung mehrerer Bilder aufeinander, d. h. die Bestimmung der Transformationen, die zwischen einzelnen Bildern einer Bildserie stattgefunden haben, könnte eine Mittelwertbildung ermöglichen. Bei der speziellen Anwendung der Fluoreszenzmessung im menschlichen Auge ist die Anwendung einer solchen Technologie jedoch nicht möglich, da die Fluoreszenzbilder aufgrund des schwachen Signals schlecht ausgesteuert und stark verrauscht sind.

Die hier vorgestellte Weiterbildung der Erfindung ist besonders für die Erstellung von Fluoreszenzmessungen des Augenhintergrundes geeignet, da sie in geeigneter Weise die Mittelung von möglicherweise stark verrauschten Bildern sich bewegender Objekte ermöglicht. In einer Vorrichtung zur Fluoreszenzmessung nach dem Stand der Technik wird am Sperrfilter der größte Teil des Lichtes, das vom zu beobachtenden Objekt ausgeht, absorbiert bzw. reflektiert.

In einer vorteilhaften Ausführungsform der Erfindung wird das Licht der Anregungslichtquelle für eine Verbesserung des Meßsignals verwendet. Dazu wird in den Beobachtungsstrahlengang ein dichroitischer Strahlteiler eingebracht, der das vom Objekt kommende Licht in den kurzwelligen Anteil des Anregungslichtes und den langwelligen Anteil der Fluoreszenzemission aufteilt. Das reflektierte Licht wird dabei auf einen zusätzlichen Sensor abgebildet.

Die Sensoren werden zeitlich synchronisiert betrieben, d. h. sie arbeiten mit gleicher Bildwiederholfrequenz und starten die Integrationszeit eines Bildes zum gleichen Zeitpunkt. Dadurch können zeitlich synchrone Bildpaare aufgenommen werden. Das Fluoreszenzbild ist dabei in der Regel schlecht ausgesteuert, während das Bild des gestreuten Anregungslichtes wesentlich besser ausgesteuert ist. Es ist daher vergleichsweise leicht, aus dem Bild des Anregungslichtes die geometrischen Transformationen, die durch die Bewegungen des Objektes zwischen den Einzelbildern einer Bildfolge entstanden sind, zu berechnen. Die inversen Transformationen werden dann auf die Fluoreszenzbilder angewendet, so dass diese durch Addition gemittelt werden können. Durch dieses Verfahren kann das Signal-zu-Rausch-Verhältnis mit jedem Bildpaar idealerweise um einen Faktor $\sqrt{2}$ verbessert werden.

In einer weiteren vorteilhaften Ausführungsform der Erfindung wird das Licht einer zusätzlichen Lichtquelle verwendet, die in einem Wellenlängenbereich emittiert, der außerhalb des Absorptions- und Emissionsbandes des fluoreszierenden Objektes liegt. Zweckmäßigerweise wird diese zusätzliche Lichtquelle in den Bleuchtungsstrahlengang eingekoppelt. In den Beobachtungsstrahlengang wird nun ein dichroitischer Strahlteiler eingebracht, der das vom Objekt kommende Licht in den kurzwelligen Anteil des Anregungslichtes und das Licht der zusätzlichen Lichtquelle einerseits und den langwelligen Anteil der Fluoreszenzemission andererseits aufteilt.

Die Verwendung einer zusätzlichen Lichtquelle bietet den Vorteil, dass unabhängig von den Anforderungen der Fluoreszenzmessung die Wellenlänge außerhalb der Absorptions- und Emissionsbanden frei gewählt werden kann und günstigerweise in einem Wellenlängenbereich gewählt wird, der eine stärkere Bestrahlung des Auges zuläßt. Durch die höhere Lichtintensität sind die Bilder, die zur Bestimmung der geometrischen Transformationen verwendet werden, besser ausgesteuert sind, dadurch kann die Mittelung der Fluoreszenzmessungen genauer ausgeführt werden.

Bevorzugte Ausführungsformen der Erfindung werden nachfolgend anhand der rein beispielhaften und keineswegs beschränkenden Zeichnungen erläutert werden. Es zeigt:

Figur 1 eine schematische Darstellung eines Ophthalmoskops des Stands der Technik;

Figur 2 eine der Figur 1 ähnliche Darstellung einer ersten Ausführungsform des erfindungsgemäßen Ophthalmoskops;

Figuren 3a bis 3c drei vereinfachte Darstellungen eines Felds von fotosensitiven Pixeln eines elektronischen Sensors zu drei verschiedenen Zeitpunkten während eines Auslesevorgangs;

Figur 4a ein vereinfachtes Zeitdiagramm zur Erläuterung der Bedeutung der Belichtungszeit $t_{INT}$ einer Pixelzeile;

Figur 4b ein vereinfachtes Zeitdiagramm eines Triggersignals zum Starten eines Auslesevorgangs des elektronischen Sensors;

Figur 5 eine schematische Darstellung einer weiteren Ausführungsform des erfindungsgemäßen Ophthalmoskops mit einer Linienfokussieroptik und einem schwenkbar gelagerten Spiegel im Beleuchtungsstrahl;

Figur 6 eine schematische Darstellung einer weiteren Ausführungsform des erfindungsgemäßen Ophthalmoskops mit zwei Beobachtungsstrahlen für stereoskopische Abbildungen;

Figur 7 eine schematische Darstellung einer weiteren Ausführungsform des erfindungsgemäßen Ophthalmoskops zur gleichzeitigen Untersuchung des Augenvordergrunds;

Figur 8 eine um Therapiefunktionen erweiterte Ausführungsform des erfindungsgemäßen Ophthalmoskops;

Figur 9a und b zwei Querschnittsdarstellungen der Pupillenebene des menschlichen Auges beim Einsatz des Ophthalmoskops aus Figur 8;

Figur 10 ein Zeitdiagramm, das die Funktionsweise der zeitlichen Aktivierung des Zielstrahls im Ophthalmoskop der Figur 8 erläutert;

Figur 11 eine schematische Ansicht, die den im Zeitdiagramm aus Figur 10 erläuterten Ablauf im Bildfeld darstellt;

Figur 12 eine Ausführungsform des erfindungsgemäßen Ophthalmoskops für die gleichzeitige Messung von Fluoreszenzemissionen; und Figur 13 eine erweiterte Ausbildung der Ausführungsform aus Figur 12, bei der eine zusätzliche Lichtquelle zur Verbesserung des Fluoreszenz-Meßergebnisses eingesetzt wird.

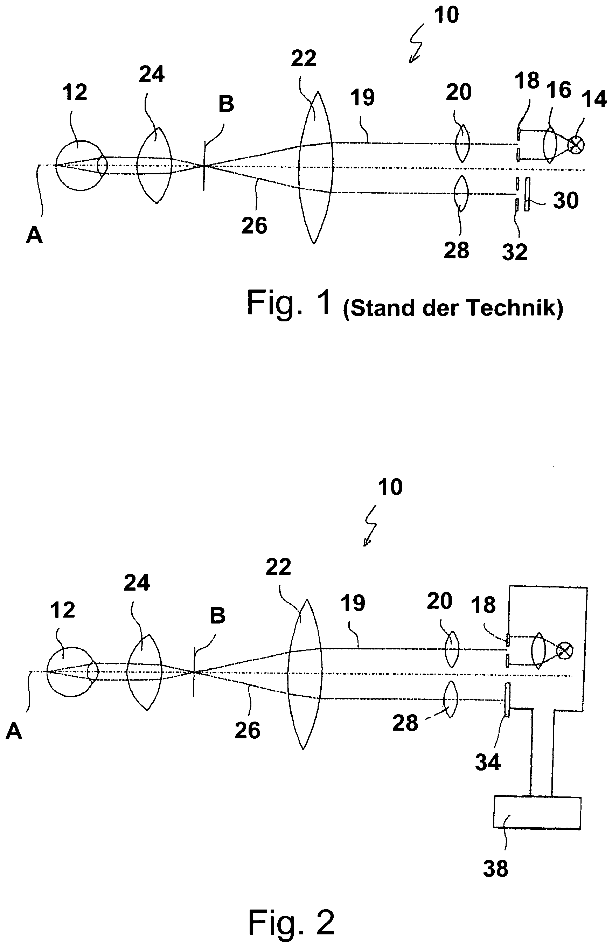

Figur 1 zeigt eine schematische Darstellung eines Ophthalmoskops 10 des Stands der Technik zur Untersuchung eines Auges 12 eines Patienten.

Das von einer Beleuchtungsvorrichtung 14 ausgesandte Licht wird mittels eines Kondensors 16 kollimiert und fällt auf eine Schlitzblende 18, die hin- und her oszilliert, und zwar in der schematischen Darstellung der Figur 1 in der Zeichenebene in einer Richtung senkrecht zur optischen Achse A des Ophthalmoskops 10. Die Schlitzblende 18 ist senkrecht zur Zeichenebene orientiert.

Auf diese Weise wird aus dem flächigen Lichtfleck, zu dem der Kondensor 16 das Licht der Beleuchtungsvorrichtung 14 kollimiert hat, eine Linie ausgekoppelt, deren Länge und Stärke von der Form der Schlitzblende 18 definiert wird, und deren Position von der aktuellen Lage der oszillierenden Schlitzblende abhängt.

Über weitere Linsen 20, 22 wird der Beleuchtungsstrahl 19 in eine Zwischenbildebene B fokussiert, aus der er über über eine ophthalmoskopische Linse 24 auf das Auge 12 abgebildet wird. Die schematische Darstellung der Figur 1 zeigt hierbei eine Abbildung auf den Augenhintergrund, beispielsweise für eine Netzhautuntersuchung.

Der aus der Reflexion des Beleuchtungsstrahls 19 am Augenhintergrund hervorgehende Beobachtungsstrahl bildet den Augenhintergrund über die ophthalmoskopische Linse 24 in die Zwischenbildebene B ab. Dieses Zwischenbild wird mittels der weiteren Linsen 22, 28 auf einen CCD-Sensor 30 fokussiert. Unmittelbar vor Erreichen des CCD-Sensors 30 muß der Beobachtungsstrahl 26 hierbei durch eine weitere Schlitzblende 32 gelangen, die vor dem CCD-Sensor synchron mit der Schlitzblende 18 im Strahlengang des Beleuchtungsstrahls 19 oszilliert.

Auf diese Weise wird mit Hilfe der Schlitzblende 32 sichergestellt, dass im wesentlichen nur der gewünschte Beobachtungsstrahl 26 den CCD-Sensor 30 erreicht, während Streulicht ausgeblendet wird. Derartiges Streulicht kann beispielsweise auf Reflexionen an der Augenvorderseite oder auch auf Streuungen an Glaskörpertrübungen des Auges 12 zurückzuführen sein.

Man erkennt in Figur 1, dass die oszillierende Schlitzblende 32 verhältnismäßig nah am CCD-Sensor 30 angeordnet ist. Dies ist unerläßlich, da mit zunehmendem Abstand zwischen Schlitzblende 32 und CCD-Sensor 30 die Unterdrückung störenden Streulichts verschlechtert wird. Allerdings führt diese Nähe dazu, dass sich Vibrationen von der oszillierenden Schlitzblende 32 auf den CCD-Sensor 30 übertragen können, beispielsweise über ein in den Figuren nicht dargestelltes Gehäuse des Ophthalmoskops 10. Dies führt zu grundsätzlichen Problemen bei der Handhabung des Ophthalmoskops 10, insbesondere verschlechtert sich die Schärfe des vom CCD-Sensor 30 gelieferten Bildes des Auges 12.

Figur 2 zeigt eine der Figur 1 ähnliche schematische Darstellung eines erfindungsgemäßen Ophthalmoskops 10, bei dem diese Probleme vermieden werden. Entsprechende Bauteile tragen in Figur 2 die gleichen Bezugszeichen wie in Figur 1. Das erfindungsgemäße Ophthalmoskop 10 gemäß der in Figur 2 dargestellten Ausführungsform unterscheidet sich von jenem des Stands der Technik dadurch, dass anstelle des CCD-Sensors 30 und der vor ihm hin- und her schwingenden Schlitzblende 32 ein elektronischer Sensor 34 im Beobachtungsstrahl 26 vorgesehen ist, der ein Feld von jeweils zeilenweise aktivierbaren und/oder auslesbaren fotosensitiven Pixeln umfaßt, die mittels einer elektronischen Steuerschaltung einzeln angesteuert werden können. Die elektronische Steuerschaltung ist im elektronischen Sensor 34 angeordnet und in den Figuren nicht dargestellt.

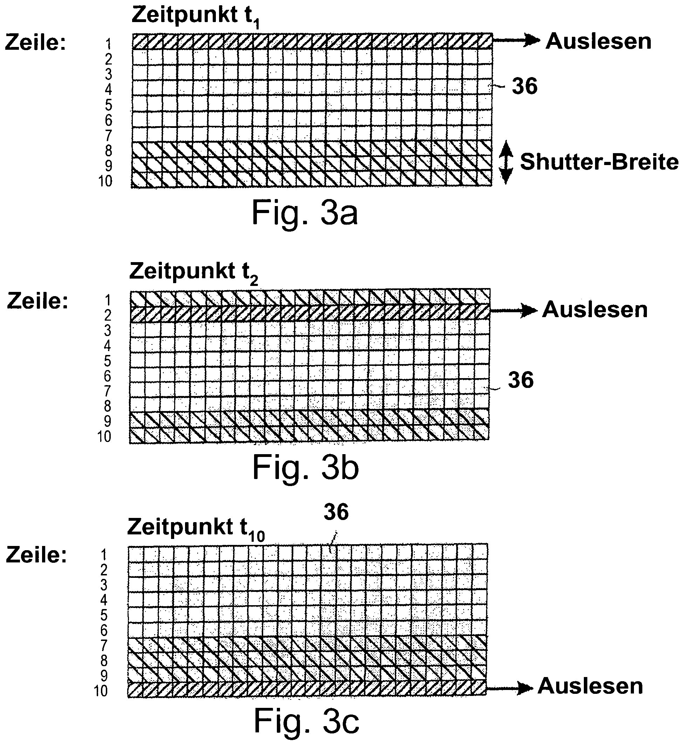

Die Funktionsweise des durch die elektronische Steuerschaltung kontrollierten elektronischen Sensors 34 wird nachfolgend anhand der Figuren 3a bis 3c erläutert werden:
Die Figuren 3a bis 3c zeigen eine schematische Vorderansicht eines Felds von fotosensitiven Pixeln eines vereinfacht dargestellten elektronischen Sensors 34. In der gezeigten vereinfachten Ausführungsform umfaßt dieses Feld von fotosensitiven Pixeln insgesamt 10 Pixelzeilen, die mit 1,2, ..., 10 durchnumeriert sind. Jede Pixelzeile umfaßt in den Figuren 3a bis 3c vierundzwanzig Pixel, die mittels fotosensitiver Elemente abhängig von der einfallenden Lichtmenge Ladungen erzeugen. Jedem Pixel 36 ist eine eigene Verstärkerelektronik zugeordnet, die die im Pixel 36 generierte elektrische Ladung in ein jeweiliges Spannungssignal umwandelt. Die Funktionsweise derartiger elektronischer Sensoren mit sogenannten "aktiven Pixeln", insbesondere CMOS-Sensoren, ist an sich bekannt und wird hier nicht weiter erläutert werden.

Mit Hilfe der im elektronischen Sensor 34 vorgesehenen elektronischen Steuerschaltung kann jede Pixelzeile in einen von drei Betriebszuständen geschaltet werden:

a) Einen deaktivierten Modus, in dem die Pixelzeile trotz Lichteinfalls keine Ladungen integriert und somit kein Spannungssignal erzeugt; in Figur 3a befinden sich die Pixelzeilen 2, 3, ..., 7 in diesem deaktivierten Modus, was durch leere Pixel 36 in diesen Pixelzeilen angedeutet wird.

b) Einen aktiven Modus, in dem Lichteinfall zur Erzeugung eines kontinuierlich ansteigenden Spannungssignals führt; in Figur 3a befinden sich die Pixelzeilen 8, 9 und 10 in diesem Betriebszustand, was durch eine einfache Schraffur dieser Pixelzeilen dargestellt ist.

c) Einen Auslesemodus, in dem eine Pixelzeile ausgelesen wird, d.h. das in einem bestimmten vorangegangenen Belichtungszeitraum aufgebaute Spannungssignal von der elektronischen Steuerschaltung abgerufen wird; die Pixelzeile wird anschließend gelöscht, d.h. das Spannungssignal wieder auf den Wert Null zurückgesetzt. In Figur 3a befindet sich nur die Pixelzeile 1 in diesem Auslesemodus, angedeutet durch die dichte Schraffur.

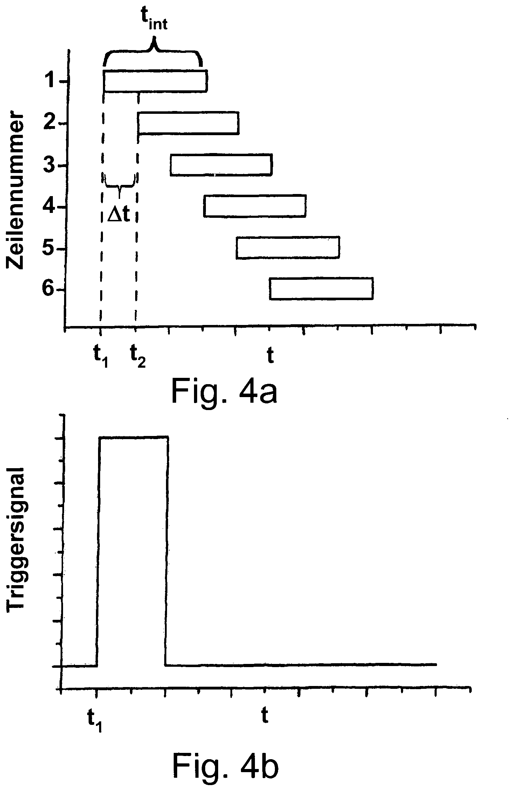

Der durch die elektronische Steuerschaltung kontrollierte Auslesevorgang des elektronischen Sensors 34 wird nachfolgend anhand der Figuren 3a bis 3c, 4a und 4b erläutert werden:
Zu einem in den Zeitdiagrammen der Figuren 4a und 4b gekennzeichneten Startzeitpunkt $t_1$ empfängt die elektronische Steuerschaltung von einer elektronischen Kontrolleinheit 38, beispielsweise einem zentralen Steuercomputer, ein Triggersignal, beispielsweise ein TTL-Signal. Die ansteigende Flanke des in Figur 4b dargestellten Triggersignals startet einen vollständigen Auslesedurchlauf des elektronischen Sensors 34, indem die Pixelzeile Nummer 1 vom inaktiven Zustand zunächst in den Auslesezustand und anschließend in den aktiven Zustand geschaltet wird, d.h. Lichteinfall führt zum Aufbau eines Spannungssignals in jedem Pixel der ersten Pixelzeile. Die erste Pixelzeile bleibt während einer Belichtungszeitdauer $t_{INT}$ in diesem aktiven Zustand und wird zu einem entsprechenden Zeitpunkt $t_1$ des nächsten Auslesedurchlaufs von der elektronischen Steuerschaltung ausgelesen.

Nach Ablauf einer einstellbaren Zeitverzögerung $\Delta t$ nach dem Startzeitpunkt $t_1$ wird auch die zweite Pixelzeile ausgelesen und in den aktiven Zustand geschaltet, und um eine weitere Zeitverzögerung $\Delta t$ später auch die dritte Pixelzeile. Bei dem in Figur 4a gezeigten Fall wird $t_{INT}=3x\Delta t$ gewählt, so dass sich zum Zeitpunkt des Auslesens und Aktivierens der vierten Pixelzeile $t_4=t_1+t_{INT}=t_1+3x\Delta t$ momentan auch die zweite und die dritte Pixelzeile im aktiven Modus befinden, wie in Figur 3a gezeigt ist, wohingegen die erste Pixelzeile gerade deaktiviert wird.

Nach dem Auslesen der ersten Pixelzeile zum Zeitpunkt $t_1$ wird diese aktiviert, und eine weitere Zeitverzögerung $\Delta t$ später, also zu einem Zeitpunkt $t_2=t_1+\Delta t$, wird die zweite Pixelzeile ausgelesen, die ab dem Zeitpunkt $t_2$ des vorhergehenden Durchlaufs während der Zeitdauer $t_{INT}$ belichtet wurde. Auf diese Weise schreitet der Auslesevorgang jeweils in zeitlichen Abständen entsprechend der Zeitverzögerung $\Delta t$ um eine Pixelzeile des elektronischen Sensors 34 weiter, bei dem in Figuren 3a bis 3c gezeigten Beispiel von Pixelzeile Nummer 1 bis zur Pixelzeile Nummer 10. Die Wahl von $t_{INT}=3x\Delta t$ führt dazu, dass zum Zeitpunkt des Auslesens der n-ten Pixelzeile drei benachbarte Pixelzeilen aktiv sind, nämlich die Pixelzeilen n-1, n-2 und n-3. Dies definiert die effektive Breite der elektronischen Schlitzblende, die sich in den Figuren 3a bis 3c gleichsam von oben nach unten über das Feld von fotosensitiven Pixeln 36 bewegt. Man beachte, dass die in einer Pixelzeile im Zeitraum $t_{INT}$ aufgebaute Spannung erst im nächsten Durchlauf der elektronischen Auslesung abgefragt wird, wenn diese Pixelzeile in den Auslesemodus geschaltet wird.

Die für einen vollständigen Durchlauf benötigte Gesamtzeit T entspricht dem Produkt aus der Zahl N der Pixelzeilen (im Beispiel der Figuren 3a bis 3c ist N=10) und der Zeitverzögerung $\Delta t$, also $T=Nx\Delta t$. Durch die Wahl der drei Parameter $\Delta t$, $t_{INT}$ und N können somit die Bildwiederholungsrate 1/T sowie die effektive Breite der elektronischen Spaltblende flexibler eingestellt werden als die entsprechenden Parameter einer mechanisch oszillierenden Schlitzblende 32 im Stand der Technik gemäß Figur 1.

Die elektronische Kontrolleinheit 38 ist dazu ausgelegt, das in Figur 4b dargestellte Triggersignal zur Synchronisierung der jeweils aktiven Pixelzeilen mit der Bewegung der Schlitzblende 18 im Beleuchtungsstrahl einzusetzen. Zum Zeitpunkt $t_1$ beispielsweise (siehe Figur 3a) sind die Pixelzeilen 8, 9 und 10 aktiv. Der Zeitpunkt $t_1$ muß daher derart gewählt sein, dass er einem Zeitpunkt entspricht, an dem die Schlitzblende 18 sich am unteren Umkehrpunkt ihrer Schwingungsbewegung befindet. Die in den Figuren 3a bis 3c und 4a dargestellte Abfolge von Pixelzeilen-Aktivierungsvorgängen von oben (in Figur 3a wird Pixelzeile Nummer 1 ausgelesen und unmittelbar anschließend aktiviert) nach unten (in Figur 3c wird Pixelzeile 10 ausgelesen und unmittelbar anschließend aktiviert) verläuft dann synchron zu einer Bewegung der Schlitzblende 18 von oben nach unten in Figur 2.

Da, wie in den Figuren 3a bis 3c und 4a gezeigt, bei diesem Prinzip des "rolling shutter" gleichsam eine elektronische Blende stets von oben nach unten über das Pixelfeld läuft, dürfen nur solche Bilder des elektronischen Sensors 34 verarbeitet werden, die einer hierzu synchronen Abwärtsbewegung des Beleuchtungsstrahls 19 entsprechen. Diejenigen Schwingungsphasen, in denen sich die Schlitzblende 18 in Figur 2 aufwärts bewegt, sind nicht verwertbar. Aus diesem Grund wird nach einem vollständigen Auslese-Durchlauf des elektronischen Sensors 34, in dem die im vorangegangenen Durchlauf in den einzelnen Pixelzeilen aufgebauten Spannungssignale ausgelesen werden, die Abgabe des nächsten Triggersignals durch die elektronische Kontrolleinheit 38 verzögert, bis die Schlitzblende 18 wieder ihren oberen Umkehrpunkt erreicht hat.

Eine weitere Verbesserung des durch den elektronischen Sensor 34 gelieferten Bilds des Auges 12 läßt sich dadurch erreichen, dass nicht nur der Auslese-Startzeitpunkt $t_1$ mit dem oberen Umkehrpunkt der schwingenden Schlitzblende 18 synchronisiert wird, sondern auch die Abwärtsbewegung der elektronischen Blende in den Figuren 3a bis 3c auf die entsprechende Abwärtsbewegung der Schlitzblende 18 im Beleuchtungsstrahl 19 abgestimmt wird. Wenn beispielsweise die Schlitzblende 18 sinusförmig im Beleuchtungsstrahl 19 hin- und her schwingt, ist ihre Geschwindigkeit, mit der sie durch den Beleuchtungsstrahl 19 fährt, am oberen und am unteren Umkehrpunkt der Schwingung jeweils gleich Null, während sie in der Mitte zwischen den beiden Umkehrpunkten ein Maximum erreicht. Dies kann im elektronischen Sensor 34 dadurch berücksichtigt werden, dass die Zeitverzögerung $\Delta t$ abweichend von Figur 4a nicht konstant gewählt wird, sondern als Funktion der jeweiligen Pixelzeile an den oberen und unteren Rändern des Pixelfelds (beispielsweise im Bereich der Pixelzeilen 1 und 10) größer gewählt wird als in der Mitte des Pixelfelds (in der Nähe der Pixelzeilen 5 und 6).

Dieser zusätzliche elektronische Aufwand, der sich ergibt, wenn man die Bewegung des "rolling shutter" des elektronischen Sensors 34 an die Bewegung der mechanischen Schlitzblende 18 anpassen möchte, läßt sich vermeiden, wenn man in umgekehrter Weise den in den Figuren 3a bis 3c, 4a und 4b dargestellten zeitlichen Ablauf der elektronischen Blende beibehält und die Bewegung der mechanischen Schlitzblende hieran anpaßt. Beispielsweise kann die elektronische Kontrolleinheit 38 einen in den Figuren nicht dargestellten Antriebsmechanismus der Schlitzblende 18 derart kontrollieren, dass die Schlitzblende 18 eine Art "Sägezahntrajektorie" durchläuft, also mit konstanter Geschwindigkeit von ihrem oberen Umkehrpunkt zu ihrem unteren Umkehrpunkt bewegt wird und anschließend deutlich schneller an ihren oberen Umkehrpunkt zurückgestellt wird.

Je nach gewünschter Bildwiederholungsfrequenz kann sich eine derartige sägezahnförmige Bewegung eines ausgedehnten Blechs, in dem Schlitzblende 18 vorgesehen ist, jedoch als schwierig erweisen.

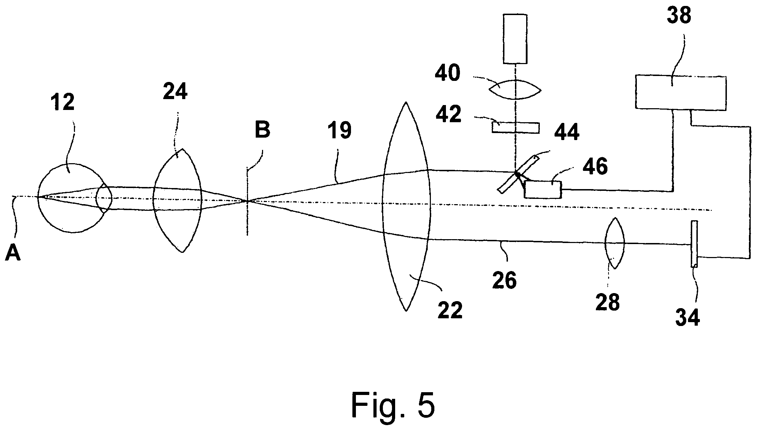

In einer in Figur 5 dargestellten bevorzugten Ausführungsform des erfindungsgemäßen Ophthalmoskops 10 ist daher in Form einer Linienfokussieroptik auch die Erzeugung des Beleuchtungsstrahls 19 gegenüber dem Stand der Technik gemäß Figur 1 abgeändert, um die Synchronisierung mit der elektronischen Blende im elektronischen Sensor 34 zu erleichtern:

In Figur 5 sind Komponenten, die jenen der in Figur 2 dargestellten Ausführungsform entsprechen, mit gleichen Bezugszeichen bezeichnet. Insbesondere wird auch bei der bevorzugten Ausführungsform gemäß Figur 5 ein elektronischer Sensor 34 mit den oben beschriebenen Eigenschaften zur Erfassung des Beobachtungsstrahls verwendet.

In dieser Ausführungsform jedoch wird als Beleuchtungsvorrichtung 14 eine punktförmige Lichtquelle, z.B. ein Laser oder das Ende eines Lichtwellenleiters eingesetzt. Das von ihm ausgehende Licht wird durch einen Kondensor 40 in ein paralleles Strahlenbündel überführt und auf eine Zylinderlinse 42 gerichtet. In Strahlrichtung hinter der Zylinderlinse 42 ist ein schwenkbar gelagerter Spiegel 44 angeordnet, der den Beleuchtungsstrahl 19 annähernd parallel zur optischen Achse A in Figur 5 nach links in Richtung auf die Linse 22 reflektiert, welche sich in der fokussierenden Ebene der Zylinderlinse 42 befindet. Die Zylinderlinse 42 ist hierbei derart positioniert, dass der von ihr erzeugte Linienfokus senkrecht zur Zeichenebene der Figur 5 orientiert ist.

Der Spiegel 44 ist schwenkbar an einer Scanvorrichtung 46 gelagert, die von der elektronischen Kontrolleinheit 38 gesteuert wird, welche gleichzeitig die elektronische Steuerschaltung des elektronischen Sensors 34 kontrolliert. Die elektronische Kontrolleinheit 38 ist dazu ausgelegt, die Scanvorrichtung 46 derart zu kontrollieren, dass sie mittels einer Drehung des Spiegels 44 in der Zeichenebene der Figur 5 den Beleuchtungsstrahl 19 synchron zur Bewegung der elektronischen Blende im elektronischen Sensor 34 über das Auge 12 scannt. Bei dem in den Figuren 3a bis 3c gezeigten kontinuierlichen linearen Abwärtsverlauf der elektronischen Blende kann der Spiegel 44 eine Sägezahntrajektorie durchlaufen, indem er beispielsweise ausgehend von der in Figur 5 gezeigten Stellung schrittweise entgegen dem Uhrzeigersinn gedreht wird, und schließlich, sobald die elektronische Blende im elektronischen Sensor 34 die untere Pixelzeile erreicht hat, beispielsweise die Pixelzeile Nummer 10 in Figur 3c, schlagartig im Uhrzeigersinn in seine Ausgangsposition gemäß Figur 5 zurückgestellt wird.

Ein derartiger schwenkbar gelagerter Spiegel weist in der Praxis ein sehr geringes Trägheitsmoment auf, so dass er nicht nur bei hohen Frequenzen, sondern generell in einem großen Frequenzbereich eingesetzt werden kann. Die Kombination eines derartigen Spiegels mit einem "rolling shutter" gemäß Figur 5, die somit eine freie Wahl der Bildwiederholrate und eine externe Synchronisation mit Hilfe der elektronischen Kontrolleinheit 38 ermöglicht, erlaubt daher eine optimale Anpassung der Beleuchtung des Auges 12 an die jeweils gewünschte Beobachtung. Durch die geringe Trägheit des Spiegels 44 werden Vibrationen im erfindungsgemäßen Ophthalmoskop 10 nahezu vollständig vermieden.

Der Einsatz einer kohärenten Lichtquelle, beispielsweise eines Lasers, erlaubt die Erzeugung eines sehr schmalen Beleuchtungsspalts. Wählt man gleichzeitig eine minimale elektronische Spaltbreite von einer einzigen Pixelzeile entprechend einer Höhe eines Pixel 36 von ca. 5 $\mu$m, so kann man eine senkrecht zur Spaltausdehnung konfokale Abbildung erzielen und somit dreidimensionale Untersuchungen des Auges 12 vornehmen.

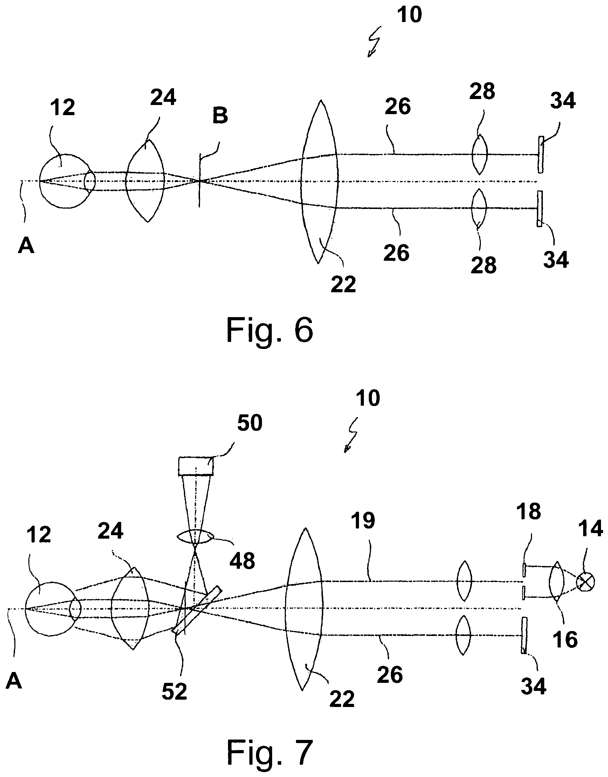

Figur 6 zeigt eine schematische Darstellung einer weiteren Ausführungsform des erfindungsgemäßen Ophthalmoskops 10 mit zwei Beobachtungsstrahlen 26 für stereoskopische Abbildungen. Der Beleuchtungsstrahlengang liegt hierbei außerhalb der Zeichenebene und ist in Figur 6 nicht dargestellt. In dieser Ausführungsform sind zwei benachbarte elektronische Sensoren 34 vorgesehen, um stereoskopische Aufnahmen des Auges 12 anzufertigen. Der Beleuchtungsstrahlengang kann hierbei entweder wie in Figur 2 gewählt sein, d.h. mit einer Schlitzblende 18, die vor einer Beleuchtungsvorrichtung 14 hin- und her oszilliert, oder wie in Figur 5 gezeigt, d.h. mit einer Linienfokussieroptik im Beleuchtungsstrahl 19. Die Vorteile der vorliegenden Erfindung, nämlich die Vermeidung mechanisch hin- und her oszillierender Schlitzblenden, kommen bei der Ausführungsform der Figur 6 besonders deutlich zum Tragen, denn bei entsprechenden Ophthalmoskopen 10 des Stands der Technik mit der Möglichkeit der stereoskopischen Beobachtung sind insgesamt drei hin- und her oszillierende Schlitzblenden erforderlich, nämlich eine Schlitzblende im Beleuchtungsstrahl 19 sowie zwei Schlitzblenden 32 in den beiden zueinander im wesentlichen parallelen Beobachtungsstrahlen. Dies führt gerade bei stereoskopischen Ophthalmoskopen 10 des Stands der Technik zu ausgeprägten Vibrationsproblemen, die durch die Erfindung vermieden werden.

Figur 7 zeigt eine weitere Ausführungsform des erfindungsgemäßen Ophthalmoskops 10 mit einer zusätzlichen Abbildungseinheit, die den Augenvordergrund durch die ophthalmoskopische Linse 24 und eine weitere Linse 48 auf einen separaten Bildsensor 50 abbildet. Beim separaten Bildsensor 50 handelt es sich vorzugsweise ebenfalls um einen elektronischen Sensor mit aktiven Pixeln, beispielsweise einen CMOS-Sensor, wie er vorstehend ausführlich beschrieben wurde.

Die Trennung des Strahlengangs von Augenhintergrund- und Augenvordergrundbeobachtung wird in dieser Ausführungsform durch einen Strahlteiler 52 vorgenommen, wobei vorzugsweise ein Folienstrahlteiler (pellicle beam splitter) zum Einsatz kommt.

Der Augenvordergrund wird in dieser Ausführungsform mit einer in Figur 7 nicht dargestellten separaten Lichtquelle beleuchtet, die direkt vor dem Auge 12 positioniert ist und idealerweise im Infraroten emittiert.

Ansonsten entspricht die Ausführungsform der Figur 7 jener der Figur 2, d.h. mit einer mechanisch oszillierenden Schlitzblende 18 im Beleuchtungsstrahl 19. Selbstverständlich kann jedoch auch bei der Ausführungsform der Figur 7 die anhand der Figur 5 erläuterte Linienfokussieroptik zum Einsatz gelangen.

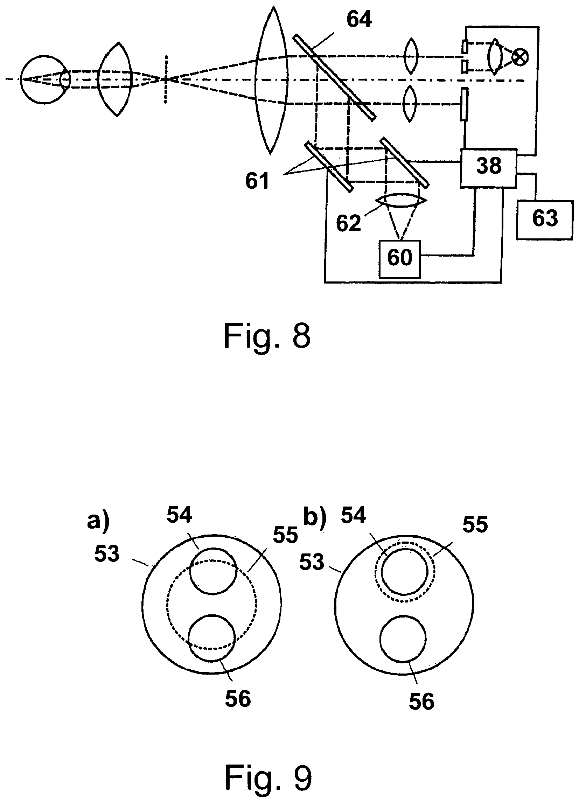

Figur 8 zeigt eine weitere Ausführungsform des erfindungsgemäßen Ophthalmoskops, die ausgehend von der Ausführungsform der Figur 2 um Therapiefunktionen erweitert worden ist. Hierzu ist eine weitere Lichtquelle 60 vorgesehen, die einen Therapie-Lichtstrahl erzeugt, welcher mittels einer Linse 62 kollimiert und über einen Strahlteiler 64 koaxial zur Hauptachse des Ophthalmoskops in den Strahlengang eingekoppelt wird. Der Therapie-Lichtstrahl wird dabei über zwei Spiegel 61 umgelenkt, die jeweils um eine zur Zeichenebene der Figur 8 senkrechte und eine in der Zeichenebene liegende Achse schwenkbar gelagert sind. Die elektronische Kontrolleinheit 38 enthält auch eine Steuereinheit, die die Schwenkbewegung der Spiegel 61 basierend auf Sollwerten kontrolliert, die ein Benutzer mittels einer Schnittstelle 63, beispielsweise eines Joysticks, eingeben kann.

Figur 9a zeigt eine schematische Querschnittsdarstellung der Pupillenebene des Auges eines Patienten im Fall der Verwendung des Ophthalmoskops aus Figur 8. Hierbei bezeichnet Bezugsziffer 53 die Pupille des Auges 12, Bezugsziffer 54 bezeichnet die Pupille des Beleuchtungsstrahlengangs, und Bezugsziffer 56 bezeichnet die Pupille des Beobachtungsstrahlengangs. Die Lage des zusätzlich mit Hilfe des Strahlteilers 64 eingekoppelten Therapie-Lichtstrahls ist in Figur 9a gestrichelt eingezeichnet und mit Bezugsziffer 55 bezeichnet. Entsprechend der Einkopplung des Therapie-Lichtstrahls in Figur 8 liegt der Therapie-Lichtstrahl 55 in Figur 9a zentral, d.h. auf der Hauptachse des erfindungsgemäßen Ophthalmoskops 10.

Alternativ kann der Therapie-Lichtstrahl mit Hilfe der Spiegel 61 und des Strahlteilers 64 auch dezentral, beispielsweise koaxial zum Beleuchtungsstrahlengang, eingekoppelt werden. Die entsprechende Querschnittsdarstellung der Pupillenebene des Patienten ist in Figur 9b gezeigt.

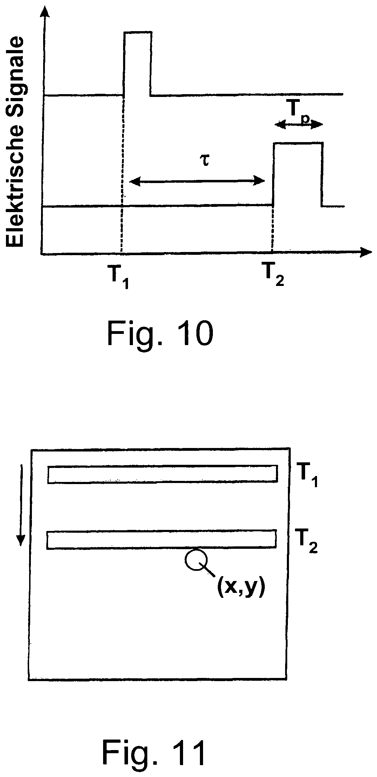

Figur 10 zeigt ein Zeitdiagramm, das die Funktionsweise der zeitlichen Aktivierung des Zielstrahls erläutert. Der Zeitpunkt $T_1$ stellt den Startpunkt der Blendenbewegung an einem Ende des Bildfeldes dar. Er entspricht zweckmäßigerweise dem oben erläuterten Zeitpunkt $t_1$, zu dem die erste Pixelzeile des elektronischen Sensors 34 ausgelesen wird. Somit ist auch der Zeitpunkt $T_1$ durch die in Figur 4b dargestellte ansteigende Flanke eines Rechteckimpulses definiert. Nach einer zeitlichen Verzögerung $\tau$, die sich wie oben beschrieben aus der Position des Zielstrahlflecks im Bildfeld (oder durch damit verbundene Größen) berechnen läßt, wird der Zielstrahl dann während eines Zeitraums $T_p$ zu einem Zeitpunkt $T_2$ aktiviert, was in Figur 10 wiederum durch einen Rechteckimpuls angedeutet ist.

Figur 11 stellt den im Zeitdiagramm der Figur 10 erläuterten Ablauf im Bildfeld dar. Zum Zeitpunkt $T_1$ befinden sich die Blenden am Startpunkt ihrer Bewegung. In Figur 11 ist der Startpunkt als am oberen Rand des Bildfeldes befindlich dargestellt. Die Bewegung der Blenden erfolgt vom Zeitpunkt $T_1$ an abwärts über das Bildfeld, wie durch einen Pfeil in Bewegungsrichtung angedeutet ist. Zum Zeitpunkt $T_2$ hat der untere Rand der Blende des erfindungsgemäßen Ophthalmoskops 10 diejenige Position (x, y) im Bildfeld erreicht, an der sich der Zielstrahlfleck befindet. Zu diesem Zeitpunkt wird daraufhin der Zielstrahl für die Zeitdauer $T_p$ aktiviert. Anders ausgedrückt wird das Einstrahlen des Zielstrahls auf diejenigen kurzen Zeiträume beschränkt, in denen die Blenden des Ophthalmoskops 10 tatsächlich die Beobachtung des gewünschten Augenbereichs erlauben. Außerhalb dieser kurzen Zeiträume ist der Zielstrahl ausgeschaltet, wodurch störende Reflexe minimiert werden. Die Synchronisation dieses gepulsten Zielstrahls mit den Blenden erfolgt wiederum mit Hilfe der elektronischen Kontrolleinheit 38.

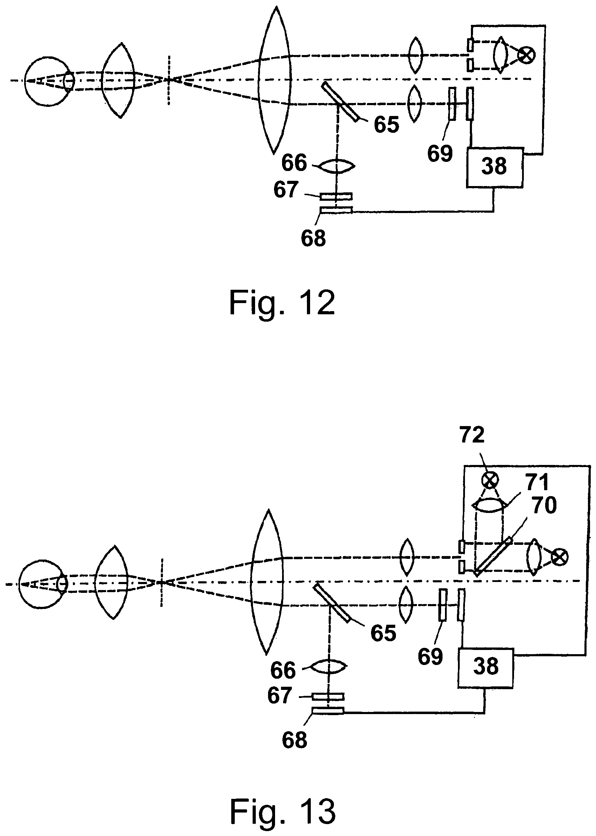

Figur 12 zeigt eine weitere Ausführungsform des erfindungsgemäßen Ophthalmoskops für die Messung von Fluoreszenzemissionen. Hierbei ist ein Strahlteiler 65 in den Beobachtungsstrahlengang eingebracht, der wellenlängenabhängig einen Teil des vom Objekt stammenden Lichts über eine Linse 66 auf einen zweiten Sensor 68 reflektiert. Der Strahlteiler 65 fungiert dabei gleichzeitig als Sperrfilter für die Fluoreszenzmessung. Die Filterwirkung des Strahlteilers kann durch zusätzliche Filter in Transmission 67 und 69 verbessert werden. Die elektronische Kontrolleinheit 38 enthält hierbei eine Steuereinheit, welche sicherstellt, dass die Sensoren 68 und 34 synchron ausgelesen werden und damit zwei Bilder zum gleichen Zeitpunkt aufgenommen werden.

Figur 13 stellt eine Weiterentwicklung der Ausführungsform der Figur 12 dar, bei der eine zusätzliche Lichtquelle 72 zur Verbesserung des Fluoreszenz-Meßergebnisses eingesetzt wird. Das Licht dieser zusätzlichen Lichtquelle 72 wird über einen Kondensor 71 kollimiert und über einen Strahlteiler 70 in den Beleuchtungsstrahlengang eingekoppelt. Die Lichtquelle 72 emittiert Licht in einem Wellenlängenbereich, der außerhalb der Absorptions- und Emissionsbanden des Fluoreszenzfarbstoffes liegt.

Die Erfindung ist nicht auf die als nicht beschränkende Beispiele vorgestellten Ausführungsformen beschränkt. So versteht sich beispielsweise, dass der anhand der Figuren 3a bis 3c erläuterte elektronische Sensor 34 nicht nur die dort aus Gründen der Einfachheit gezeigten zehn Pixelzeilen aufweist, sondern in der Praxis mehr als tausend Pixelzeilen.

Ferner versteht sich, dass der konkrete Verlauf der Strahlengänge des Beleuchtungsstrahls 19 sowie des Beobachtungsstrahls 26 bzw. der Beobachtungsstrahlen 26 im Falle stereoskopischer Abbildungen durch bekannte optische Elemente variiert werden kann. Ebenso versteht es sich, dass weitere Lichtstrahlen in die vorgestellten Strahlengänge eingekoppelt werden können, beispielsweise zu Therapie- oder Diagnosezwecken, wie es aus dem Stand der Technik grundsätzlich bekannt ist.
In all diesen abgewandelten Ausführungsformen bietet die Erfindung nicht nur die oben erläuterten Vorteile einer deutlichen Verringerung bzw. vollständigen Beseitigung mechanischer Vibrationen aufgrund hin- und her schwingender Schlitzblenden, sondern zahlreiche weitere Vorteile:

a) Wie bereits betont wurde, befindet sich die elektronische Blende beim elektronischen Sensor 34 in dessen Bildebene, so dass die Ausblendung von Streulicht optimiert wird.

b) Kontinuierliche Bildgebungsverfahren setzen eine ausreichend hohe Bildwiederholungsfrequenz von mehr als 10Hz voraus, um einen flüssigen Bewegungseindruck beim Beobachter zu erzeugen. Die im Stand der Technik eingesetzten Schlitzblenden müssen also mit hoher Frequenz schwingen, was in der Praxis nur mit einem mechanischen Oszillator in Resonanz zu erreichen ist. Dies setzt wiederum eine exakte und auf geringe Abweichungen äußerst empfindliche Abstimmung der Eigenfrequenz des Oszillators mit der Bildwiederholungsfrequenz des CCD-Sensors 30 voraus. Somit ist die Bildwiederholfrequenz für einen gegebenen Oszillator fest. Beim erfindungsgemäßen Ophthalmoskop 10 hingegen, insbesondere in der Ausführungsform gemäß Figur 5, die ohne jegliche mechanische hin- und her schwingende Schlitzblende arbeitet, ist keine derartige Beschränkung durch eine Resonanzbedingung gegeben.

c) Beim Ophthalmoskop des Stands der Technik ist die Spaltbreite für eine gegebene mechanisch schwingende Blende unveränderlich. Viele Anwendungen verlangen jedoch nach einer feinstufigen Variation der Spaltbreite und damit der effektiven Belichtungszeit. Beispielsweise ist eine Variation der Schlitzbreite oder der Bildwiederholfrequenz und damit der effektiven Belichtungszeit wünschenswert, um auf unterschiedliche Anwendungsfälle einzugehen: bei Angiographie des Augenhintergrunds muß die schwache Fluoreszenzemission eines Farbstoffes detektiert werden, was eine möglichst lange Belichtungszeit erfordert. Dies ist durch Einstellen der Parameter des elektronischen Sensors 34, insbesondere die Parameter N, $\Delta t$ und $t_{INT}$, problemlos möglich.

d) Beim Ophthalmoskop des Stands der Technik beträgt die Frequenz des Oszillators für den Antrieb der Schlitzblende in der Praxis ein Vielfaches der Bildwiederholfrequenz des CCD-Sensors, da mechanische Oszillatoren mit geringer Frequenz zum einen ungenau in ihrer Schwingfrequenz abzustimmen sind und zum anderen auch zu besonders unangenehmen Vibrationen des gesamten Gerätes führen. Dies führt zu Doppelbelichtungen auf dem CCD-Sensor 30 und damit zu Bildunschärfe und Bewegungsartefakten durch schnelle Bewegung des Auges 12 oder sonstigen untersuchten Objekts. Die Verwendung von Bildsensoren, die nach einem der gängigen Video-Standards (PAL, NTSC) zwei Halbbilder, die zu unterschiedlichen Zeitpunkten aufgenommen werden, zu einem Vollbild zusammenfügen (Interlacing), hat sich als besonders nachteilig erwiesen. Die weitere computergestützte Bearbeitung und Auswertung der Bilder (z.B. die Bestimmung von Lage, Größe, Form etc. von Blutgefäßen im Auge 12) wird durch derartige Bewegungsartefakte oder Interlacing-Artefakte erschwert. Die erfindungsgemäße Vermeidung der mechanischen Schlitzblende vor dem Sensor löst all diese Probleme.

e) Der in Resonanz schwingende mechanische Oszillator zum Antrieb der Schlitzblenden beim Ophthalmoskop des Stands der Technik führt in der Regel eine sinusförmige Bewegung aus. Die integral während einer Periode durch die schwingende Blende auf das Auge 12 fallende Lichtmenge ist daher ungleichmäßig über das Auge 12 verteilt und führt zu einer ungleichmäßigen Kontrast- und Helligkeitsverteilung im Bild. Um dennoch eine gleichmäßige Ausleuchtung des Bildausschnitts zu erhalten, wird die Amplitude der Schwingung häufig so weit erhöht, dass nur der näherungsweise lineare Anteil der Blendentrajektorie im Sichtfeld liegt. Dadurch wird jedoch nur ein Bruchteil des auf das Auge 12 fallenden Lichts zur Bildgebung benutzt, was zu einer Verringerung der Sensitivität des Ophthalmoskops 10 führt. Beim erfindungsgemäßen Ophthalmoskop 10 hingegen wird das verfügbare Licht wesentlich besser ausgenutzt.

f) Durch die Verwendung einer herkömmlichen Glühlampe oder Halogenlampe, die die Blendenebene des Beleuchtungsstrahlenganges gleichmäßig ausleuchtet, wird die verfügbare Leuchtkraft der Beleuchtungsvorrichtung 14 nur mangelhaft ausgenutzt. Die Wärmeabstrahlung der Beleuchtungsvorrichtung 14 führt zu einer weiteren Beeinträchtigung der Handhabung des Gerätes. Diese Probleme lassen sich bei der in Figur 5 gezeigten Ausführungsform mit einem Laser und einer Linienfokussieroptik vermeiden.

g) Eine Halogenlampe, wie sie im Stand der Technik eingesetzt wird, strahlt ein breites, thermisches Frequenzspektrum ab, dessen Maximum im Infrarotbereich liegt, und das zu kürzeren Wellenlängen hin abfällt. Eine solche Lichtquelle ist dafür ungeeignet, ein kontrastreiches Farbbild des Augenhintergrunds zu erzeugen, da der Augenhintergrund hauptsächlich im roten Spektralbereich reflektiert. Halbleiterbasierte Bildsensoren sind zudem stärker im roten und infraroten Spektralbereich empfindlich als bei kürzeren Wellenlängen. Ein mit Halogenbeleuchtung aufgenommenes Farbbild des Augenhintergrunds hat daher einen dominanten Rot-Kanal, jedoch nur schwach ausgesteuerte Grün- und Blau-Kanäle. Das Bild ist daher verrauscht und kontrastarm. Der Einsatz von Filtern zur Beeinflussung des Beleuchtungsspektrums verbessert zwar den Farbeindruck, reduziert jedoch stark die Leuchtkraft der Lichtquelle. Für die bereits angesprochene Beobachtung von Fluoreszenzemissionen ist aber eine intensive Anregung der Farbstoffe in einem engen Spektralbereich notwendig. Dies können in der Ausführungsform gemäß Figur 5 moderne Lichtquellen wie z.B. LEDs oder Laser leisten.

Die Erfindung betrifft unter Umständen folgende Aspekte:

1. Ophthalmoskop (10) zur Untersuchung eines Auges (12) eines Patienten, umfassend:

eine Beleuchtungsvorrichtung (14) zur Erzeugung eines Beleuchtungsstrahls (19);
eine Beleuchtungs-Abbildungsoptik zum Abbilden des Beleuchtungsstrahls (19) auf das Auge (12), und
Mittel (18) zum Scannen des Beleuchtungsstrahls (19) über das Auge (12), sowie
eine Beobachtungsvorrichtung (30), die einen elektronischen Sensor (34) mit einem Feld von fotosensitiven Pixeln (36) umfaßt, die jeweils zeilenweise aktivierbar und/oder auslesbar sind;
eine Beobachtungs-Abbildungsoptik zum Abbilden eines durch Reflexion des Beleuchtungsstrahls (19) am Auge (12) erzeugten Beobachtungsstrahls auf die Beobachtungsvorrichtung (30), und
Mittel (32) zum Ausblenden von Streulicht aus dem Beobachtungsstrahl,
dadurch gekennzeichnet, dass
die Mittel (32) zum Ausblenden von Streulicht aus dem Beobachtungsstrahl eine elektronische Steuerschaltung zum Auslesen wenigstens einer Pixelzeile des Sensors umfassen, und dass das Ophthalmoskop (10) eine elektronische Kontrolleinheit (38) umfaßt, die dazu ausgelegt ist, die Mittel (18) zum Scannen und die elektronische Steuerschaltung derart zu kontrollieren, dass das Scannen des Beleuchtungsstrahls (19) über das Auge (12) synchron mit der Änderung der aktuell auszulesenden Pixelzeile erfolgt.

2. Ophthalmoskop (10) nach Aspekt 1, dadurch gekennzeichnet, dass die elektronische Steuerschaltung dazu ausgelegt ist, jeweils eine einzelne Pixelzeile auszulesen.

3. Ophthalmoskop (10) nach Aspekt 2, dadurch gekennzeichnet, dass die elektronische Steuerschaltung dazu ausgelegt ist, jeweils nach Ablauf einer einstellbaren Zeitverzögerung ($\Delta$t) die aktuell auszulesende Pixelzeile zu ändern, insbesondere von einer Pixelzeile zu einer benachbarten Pixelzeile.

4. Ophthalmoskop (10) nach einem der Aspekte 1 bis 3, dadurch gekennzeichnet, dass die elektronische Steuerschaltung ferner dazu ausgelegt ist, jede Pixelzeile während einer einstellbaren Belichtungszeit ($t_{INT}$) vor ihrem Auslesen zu aktivieren.

5. Ophthalmoskop (10) nach einem der Aspekte 1 bis 4, dadurch gekennzeichnet, dass die elektronische Steuerschaltung dazu ausgelegt ist, den Auslesevorgang infolge eines externen Triggersignals zu starten.

6. Ophthalmoskop (10) nach einem der vorhergehenden Aspekte, dadurch gekennzeichnet, dass die Mittel (18) zum Scannen des Beleuchtungsstrahls (19) über das Auge (12) einen schwenkbar gelagerten Spiegel (44) mit einer Scanvorrichtung umfassen.

7. Ophthalmoskop (10) nach Aspekt 6, dadurch gekennzeichnet, dass es ferner eine feste Spaltblende zum Auskoppeln eines linienförmigen Beleuchtungsstrahls (19) umfaßt.

8. Ophthalmoskop (10) nach Aspekt 6, dadurch gekennzeichnet, dass es ferner eine Linienfokussieroptik (40, 42) zum Fokussieren des Beleuchtungsstrahls (19) in einer Linie umfaßt.

9. Ophthalmoskop (10) nach Aspekt 8, dadurch gekennzeichnet, dass die Linienfokussieroptik (40, 42) eine Zylinderlinse (42) umfaßt.

10. Ophthalmoskop (10) nach einem der Aspekte 6 bis 9, dadurch gekennzeichnet, dass die elektronische Kontrolleinheit (38) dazu ausgelegt ist, die elektronische Steuerschaltung des elektronischen Sensors (34) und die Scanvorrichtung des schwenkbaren Spiegels (44) zu kontrollieren.

11. Ophthalmoskop (10) nach Aspekt 10, dadurch gekennzeichnet, dass die Stärke der Linie des fokussierten Beleuchtungsstrahls (19) der Höhe einer Pixelzeile des elektronischen Sensors (34) entspricht.

12. Ophthalmoskop (10) nach einem der Aspekte 8 bis 11, dadurch gekennzeichnet, dass die Beleuchtungsvorrichtung (14) ein Laser ist.

13. Ophthalmoskop (10) nach einem der vorhergehenden Aspekte, dadurch gekennzeichnet, dass die Beobachtungsvorrichtung (30) zwei elektronische Sensoren (34) zur stereoskopischen Untersuchung des Auges (12) umfaßt.

14. Ophthalmoskop (10) nach einem der vorhergehenden Aspekte, dadurch gekennzeichnet, dass es ferner eine separate Lichtquelle und einen separaten Bildsensor (50) zur Untersuchung des Auges (12) umfaßt.

15. Ophthalmoskop (10) nach einem der vorhergehenden Aspekte, dadurch gekennzeichnet, dass es ferner Mittel zur Durchführung einer weiteren Abbildung auf den Hintergrund des Auges (12) in einem separaten Strahlengang umfaßt.

16. Ophthalmoskop (10) nach Aspekt 15, dadurch gekennzeichnet, dass es einen Spiegel umfaßt, der derart angeordnet ist, dass die Einkopplung eines zusätzlichen Strahls in dem separaten Strahlengang nicht koaxial zum Beobachtungsstrahl (26) erfolgt.

17. Ophthalmoskop (10) nach Aspekt 16, dadurch gekennzeichnet, dass der Spiegel derart angeordnet ist, dass die Einkopplung des zusätzlichen Strahls ferner nicht koaxial zum Beleuchtungsstrahl (19) erfolgt.

18. Ophthalmoskop (10) nach einem der Aspekte 14 bis 17, dadurch gekennzeichnet, dass der separate Strahlengang koaxial zur Hauptachse des Ophthalmoskops (10) ist.

19. Ophthalmoskop (10) nach einem der Aspekte 15 bis 18, dadurch gekennzeichnet, dass es einen wellenlängenabhängigen Strahlteiler (64) zur Einkopplung eines zusätzlichen Strahls umfaßt.

20. Ophthalmoskop (10) nach einem der Aspekte 15 bis 18, dadurch gekennzeichnet, dass es einen wellenlängenunabhängigen Strahlteiler zur Einkopplung eines zusätzlichen Strahls sowie einen Sperrfilter im Beobachtungsstrahl (26) umfaßt.

21. Ophthalmoskop (10) nach einem der Aspekte 15 bis 20, dadurch gekennzeichnet, dass es eine polarisierte Lichtquelle (60) zur Erzeugung eines zusätzlichen Lichtstrahls sowie einen polarisationsabhängigen Strahlteiler (64) zu seiner Einkopplung umfaßt.

22. Ophthalmoskop (10) nach Aspekt 21, dadurch gekennzeichnet, dass es ferner einen oder mehrere schwenkbare Spiegel (61) umfaßt, die derart zwischen der Lichtquelle (60) und dem Strahlteiler (64) angeordnet sind, dass durch Verkippung eines oder mehrerer der Spiegel (61) um jeweils eine oder mehrere Achsen eine Translation des Abbildes auf dem Hintergrund des Auges (12) erzielbar ist.

23. Ophthalmoskop (10) nach Aspekt 22, dadurch gekennzeichnet, dass es eine Steuereinheit (38) zur Steuerung der Spiegel (61) umfaßt.

24. Ophthalmoskop (10) nach Aspekt 23, dadurch gekennzeichnet, dass die Steuereinheit dazu ausgelegt ist, aus einer Größe, die direkt mit der Position des durch den zusätzlichen Lichtstrahl erzeugten Flecks im Bildfeld zusammenhängt, und dem Startzeitpunkt ($T_1$) der Blendenbewegung ein Zeitfenster ($T_p$) zu bestimmen, in dem der zusätzliche Lichtstrahl angeschaltet wird.

25. Ophthalmoskop (10) nach einem der Aspekte 22 bis 24, dadurch gekennzeichnet, dass es ferner eine mit der Steuereinheit (38) verbundene Schnittstelle (63) zur manuellen Steuerung der Position des Abbildes durch einen Benutzer umfaßt.

26. Ophthalmoskop (10) nach einem der vorhergehenden Aspekte, dadurch gekennzeichnet, dass es einen vor dem Sensor (34) angeordneten Sperrfilter umfaßt, der in Verbindung mit einer geeigneten Lichtquelle im Beleuchtungsstrahlengang (19) die Beobachtung von Fluoreszenzemissionen vom Hintergrund des Auges (12) ermöglicht.

27. Ophthalmoskop (10) nach einem der Aspekte 1 bis 25, dadurch gekennzeichnet, dass es einen dichroitischen Strahlteiler (65) und einen zusätzlichen Sensor (68) umfaßt, die in Verbindung mit einer geeigneten Lichtquelle im Beleuchtungsstrahlengang (19) die Beobachtung von Fluoreszenzemissionen vom Hintergrund des Auges (12) ermöglichen.

28. Ophthalmoskop (10) nach Aspekt 27, dadurch gekennzeichnet, dass es zusätzlich zur Beleuchtungsvorrichtung (14) eine weitere Lichtquelle (72) umfaßt, deren Licht zur Anregung von Fluoreszenzemissionen am Hintergrund des Auges (12) in den Beobachtungsstrahlengang (19) eingekoppelt wird.

29. Ophthalmoskop (10) nach einem der Aspekte 26 bis 28, dadurch gekennzeichnet, dass die elektronische Kontrolleinheit (38) dazu ausgelegt ist, aus einer mit dem Sensor (34, 68) aufgenommenen Bildfolge Transformationen zwischen den Einzelbildern zu berechnen und diese Transformationen auf die mit dem Sensor (68, 34) aufgenommenen Bilder anzuwenden und die Bilder zu mitteln.

**Patentansprüche**

1. Ophthalmoskop (10), umfassend:

   - eine Beleuchtungsvorrichtung (14) zur Erzeugung eines Beleuchtungsstrahls (19),
   - eine Beleuchtungs-Abbildungsoptik zum Abbilden des Beleuchtungsstrahls (19) auf ein Auge (12),
   - eine Beobachtungsvorrichtung (30) mit einem lichtempfindlichen Sensor (34) zum Erfassen von Bildern des Auges,
   - eine Beobachtungs-Abbildungsoptik zum Abbilden eines durch Reflexion des Beleuchtungsstrahls (19) am Auge (12) erzeugten Beobachtungsstrahls auf den Sensor (34),
   - eine Anzeige zum Anzeigen der mit dem Sensor erfassten Bilder als Live-Bild des Auges,
   - eine Therapiestrahlquelle (60) zur Applikation von Therapielichtimpulsen in dem Auge, **gekennzeichnet durch**
   - eine Steuereinheit (38), die eingerichtet ist, die mit dem Sensor (34) erfassten Bilder des Auges in Echtzeit auszuwerten und hierbei in den Bildern ein auf dem Auge ortsfestes objektbezogenes Koordinatensystem anhand von Strukturen im Auge zu bestimmen, wobei die Steuereinheit (38) außerdem eingerichtet ist, in einem weiteren Bild des Auges, das zu einem früheren Zeitpunkt oder unter Verwendung anderer Bildgebungsverfahren mit einem anderen Instrument aufgenommen worden ist, das gleiche objektbezogene Koordinatensystem anhand von Strukturen im Auge zu bestimmen und das Live-Bild mit dem weiteren Bild zu überlagern unter Zugrundelegung des gleichen objektbezogenen Koordinatensystems, wobei die Steuereinheit (38) eingerichtet ist, Koordinaten von Einwirkungsorten der applizierten Therapielichtimpulse in dem objektbezogenen Koordinatensystem zu speichern.

2. Ophthalmoskop (10) aus Anspruch 1, **dadurch gekennzeichnet, dass** die Steuereinheit (38) eingerichtet ist, Parameter der Therapielichtimpulse zu speichern, insbesondere eine Pulsdauer, eine Leistung, eine Wellenlänge und/oder eine Spotgröße der Therapielichtimpulse.

3. Ophthalmoskop (10) nach einem der vorangehenden Ansprüche, **dadurch gekennzeichnet, dass** die Steuereinheit (38) eingerichtet ist, die Informationen über die Therapielichtimpulse kurz vor, während oder nach der Applikation der Therapielichtimpulse zu speichern.

4. Ophthalmoskop (10) nach einem der vorangehenden Ansprüche, **dadurch gekennzeichnet, dass** die Steuereinheit (38) eingerichtet ist, die gespeicherten Informationen mittels einer Anzeige des Ophthalmoskops (10) sichtbar zu machen durch Überlagerung der Bilder des Auges mit den gespeicherten Informationen.

5. Ophthalmoskop (10) nach Anspruch 4, **dadurch gekennzeichnet, dass** die Steuereinheit (38) eingerichtet ist, beim Überlagern der Bilder mit den Informationen das gleiche objektbezogene Koordinatensystem den Bildern und Positionen der überlagerten Informationen zugrunde zu legen.

6. Ophthalmoskop (10) nach Anspruch 5, **dadurch gekennzeichnet, dass** die Steuereinheit (38) eingerichtet ist, die Bilder zu stabilisieren durch eine Fixierung des objektbezogenen Koordinatensystems an ein Koordinatensystem der Anzeige.

7. Ophthalmoskop (10) nach einem der vorangehenden Ansprüche, **dadurch gekennzeichnet, dass** die Steuereinheit (38) eingerichtet ist, die von dem Ophthalmoskop (10) erfassten Bilder zu überlagern mit vom Benutzer zu einem anderen Zeitpunkt erstellten graphischen Darstellungen im objektbezogenen Koordinatensystem.

8. Ophthalmoskop (10) nach einem der vorangehenden Ansprüche, **dadurch gekennzeichnet, dass** die Steuereinheit (38) zum Steuern der Therapiestrahlquelle (60) unter Verwendung von Steuerparametern und zum Speichern der Steuerparameter eingerichtet ist, wobei die Steuerparameter eine Pulsdauer, eine Pulswiederholrate und/oder eine Lichtleistung umfassen.

9. Ophthalmoskop (10) nach einem der vorangehenden Ansprüche, **dadurch gekennzeichnet, dass** die Steuereinheit (38) eingerichtet ist, die mit dem Sensor (34) erfassten Bilder mit in dem objektbezogenen Koordinatensystem definierten Zielpositionen und/oder Verbotszonen für die Therapiestrahlimpulse zu überlagern.

10. Ophthalmoskop (10) nach Anspruch 9, **dadurch gekennzeichnet, dass** die Zielpositionen und/oder die Verbotszonen zuvor von einem Benutzer definiert worden sind oder dass die Zielpositionen und/oder die Verbotszonen zuvor automatisch von der Steuereinheit (38) mittels einer Bildanalyse definiert worden sind.

11. Ophthalmoskop (10) nach einem der Ansprüche 9 oder 10, **dadurch gekennzeichnet, dass** die Steuereinheit (38) eingerichtet ist, eine Auslösung der Therapielichtimpulse freizugeben unter der Bedingung, dass ein mittels einer Zielstrahlquelle des Ophthalmoskops (10) erzeugter Zielstrahl auf eine der Zielpositionen ausgerichtet ist, und/oder die Auslösung der Therapielichtimpulse zu verhindern unter der Bedingung, dass der Zielstrahl auf eine der Verbotszonen gerichtet ist.

## Claims

1. Ophthalmoscope (10), comprising:

   - an illumination device (14) for generating an illumination beam (19),
   - illumination imaging optics for imaging the illumination beam (19) onto an eye (12),
   - an observation device (30) with a light-sensitive sensor (34) for capturing images of the eye,
   - observation imaging optics for imaging an observation beam, generated by reflection of the illumination beam (19) at the eye (12), onto the sensor (34),
   - a display for displaying the images taken by the sensor as live image of the eye,
   - a therapy beam source (60) for application of therapy light pulses in the eye,
   **characterized by**
   - a control unit (38), which is configured to evaluate the images of the eye captured with the sensor (34) in real-time and thereby determine in the images a object-related coordinate system which is stationary on the eye on the basis of structures in the eye, wherein the control unit (38) is further configured to determine in a further image of the eye, which has been captured at an earlier instance of time or by using other imaging methods with another instrument, the same object-related coordinate system on the basis of structures in the eye and to superimpose the live image with the further image on the basis of the same object-reference coordinate system, wherein the control unit (38) is configured to store coordinates of affected locations of the applied therapy light pulses in the object-related coordinate system.

2. Ophthalmoscope (1) of claim 1, **characterized in that** the control unit (38) is configured to store parameters of the therapy light pulses, particularly a pulse duration, a light power, a wavelength and/or a spot size of the therapy light pulses.

3. Ophthalmoscope (10) according to one of the preceding claims, **characterised in that** the control unit (38) is configured to store the information about the therapy light pulses shortly before, during or after the application of the therapy beam pulses.

4. Ophthalmoscope (10) according to one of the preceding claims, **characterised in that** the control unit (38) is configured to display the stored information by means of a display of the ophthalmoscope (10) by superimposing the images of the eye with the stored information.

5. Ophthalmoscope (10) according to claim 4, **characterised in that** the control unit (38) is configured to take the same object-related coordinate system as a basis for the images and positions of the superimposed information when superimposing the images with the information.

6. Ophthalmoscope (10) according to claim 5, **characterised in that** the control unit (38) is configured to stabilize the images by fixing the object-related coordinate system to a coordinate system of the display.

7. Ophthalmoscope (10) according to one of the preceding claims, **characterised in that** the control unit (38) is configured to superimpose the images captured by the ophthalmoscope (10) with graphical representations produced by the user at another instant of time in the object-related coordinate system.

8. Ophthalmoscope (10) according to one of the preceding claims, **characterised in that** the control unit (38) is configured to control the therapy beam source (60) using control parameters and to store the control parameters, wherein the control parameters include a pulse dusration, a pulse repetition rate and/or a light power.

9. Ophthalmoscope (10) according to one of the preceding claims, **characterised in that** the control unit (38) is configured to superimpose the images captured by the sensor (34) with target positions defined in the object-related coordinate system and/or prohibited zones for the therapy beam pulses.

10. Ophthalmoscope (10) according to claim 9, **characterised in that** the target positions and/or the prohibited zones have been previously defined by a user or that the target positions and/or the prohibited zones have been previously defined in an automatic manner by the control unit (38) by means of a image analysis.

11. Ophthalmoscope (10) according to one according to claims 9 or 10, **characterized in that** the control unit (38) is configured to enable triggering the therapy light pulses under the condition that an aiming beam generated by an aiming beam source of the ophthalmoscope (10) is directed onto a target position and/or to prevent triggering the therapy light pulses under the condition that the aiming beam is directed onto one of the prohibited zones.

## Revendications

1.  Ophtalmoscope (10) comprenant :

    - un dispositif d'éclairage (14) pour la production d'un faisceau d'éclairage (19),
    - une optique de représentation d'éclairage pour la représentation du faisceau d'éclairage (19) sur un oeil (12),
    - un dispositif d'observation (30) avec un capteur photosensible (34) pour la capture d'images de l'oeil,
    - une optique de représentation d'observation pour la représentation sur le capteur (34) d'un faisceau d'observation généré par la réflexion du faisceau d'éclairage (19) sur l'oeil (12),
    - un affichage pour l'affichage des images capturées avec le capteur sous la forme d'une image en direct de l'oeil,
    - une source de faisceaux thérapeutiques (60) pour l'application d'impulsions lumineuses thérapeutiques dans l'oeil,
    **caractérisé par**
    - une unité de commande (38) qui est conçue pour analyser les images de l'oeil capturées avec le capteur (34) en temps réel et pour en déduire, dans les images, un système de coordonnées lié à un objet, fixe sur l'oeil, à l'aide de structures dans l'oeil, l'unité de commande (38) étant en outre conçue pour déterminer, dans une autre image de l'oeil, qui a été prise à un moment antérieur ou à l'aide d'autres procédés d'imagerie avec un autre instrument, le même système de coordonnées lié à un objet à l'aide de structures dans l'oeil, et pour superposer l'image en direct avec l'autre image sur la base du même système de coordonnées lié à un objet, l'unité de commande (38) étant conçue pour enregistrer les coordonnées des lieux d'action des impulsions lumineuses thérapeutiques appliquées dans le système de coordonnées lié à un objet.

2.  Ophtalmoscope (10) selon la revendication 1, **caractérisé en ce que** l'unité de commande (38) est conçue pour enregistrer des paramètres des impulsions lumineuses thérapeutiques, plus particulièrement une durée d'impulsion, une puissance, une longueur d'onde et/ou une taille de point des impulsions lumineuses thérapeutiques.

3.  Ophtalmoscope (10) selon l'une des revendications précédentes, **caractérisé en ce que** l'unité de commande (38) est conçue pour enregistrer les informations concernant les impulsions lumineuses thérapeutiques un peu avant, pendant ou après l'application des impulsions lumineuses thérapeutiques.

4.  Ophtalmoscope (10) selon l'une des revendications précédentes, **caractérisé en ce que** l'unité de commande (38) est conçue pour rendre visibles les informations enregistrées au moyen d'un affichage de l'ophtalmoscope (10) par superposition des images de l'oeil avec les informations enregistrées.

5.  Ophtalmoscope (10) selon la revendication 4, **caractérisé en ce que** l'unité de commande (38) est conçue pour établir, lors de la superposition des images avec les informations, le même système de coordonnées lié à un objet pour les images et les positions des informations superposées.

6.  Ophtalmoscope (10) selon la revendication 5, **caractérisé en ce que** l'unité de commande (38) est conçue pour stabiliser les images par une fixation du système de coordonnées lié à un objet à un système de coordonnées de l'affichage.

7.  Ophtalmoscope (10) selon l'une des revendications précédentes, **caractérisé en ce que** l'unité de commande (38) est conçue pour superposer les images capturées par l'ophtalmoscope (10) avec les représentations graphiques créées à un autre moment par l'utilisateur dans le système de coordonnées lié à un objet.

8.  Ophtalmoscope (10) selon l'une des revendications précédentes, **caractérisé en ce que** l'unité de commande (38) est conçue pour la commande de la source de faisceaux thérapeutiques (60) à l'aide de paramètres de commande et pour l'enregistrement des paramètres de commande, les paramètres de commande comprenant une durée d'impulsion, un taux de répétition des impulsions et/ou une puissance lumineuse.

9.  Ophtalmoscope (10) selon l'une des revendications précédentes, **caractérisé en ce que** l'unité de commande (38) est conçue pour superposer les images capturées avec le capteur (34), avec des positions cibles définies dans le système de coordonnées lié à un objet et/ou avec des zones interdites pour les impulsions de faisceaux thérapeutiques.

10. Ophtalmoscope (10) selon la revendication 9, **caractérisé en ce que** les positions cibles et/ou les zones interdites ont été définies auparavant par un utilisateur ou **en ce que** les positions cibles et/ou les zones interdites ont été définies auparavant automatiquement par l'unité de commande (38) au moyen d'une analyse d'image.

11. Ophtalmoscope (10) selon l'une des revendications 9 ou 10, **caractérisé en ce que** l'unité de commande (38) est conçue pour permettre un déclenchement des impulsions lumineuses thérapeutiques si un faisceau cible généré au moyen d'une source de faisceau cible de l'ophtalmoscope (10) est orienté vers une des positions cibles et/ou pour empêcher le déclenchement des impulsions lumineuses thérapeutiques si le faisceau cible est dirigé vers une des zones interdites.

Fig. 1 (Stand der Technik)

Fig. 2

**Zeitpunkt t₁**

Zeile:

Auslesen

36

Shutter-Breite

## Fig. 3a

**Zeitpunkt t₂**

Zeile:

Auslesen

36

## Fig. 3b

36

**Zeitpunkt t₁₀**

Zeile:

Auslesen

## Fig. 3c

Fig. 4a

Fig. 4b

Fig. 5

**Fig. 6**

**Fig. 7**

Fig. 8

Fig. 9

Fig. 10

Fig. 11

Fig. 12

Fig. 13

**EP 2 386 244 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6267756 B1
- WO 02094088 A
- EP 1389943 B1
- WO 9905853 A
- US 6758564 B2